(19) Europäisches Patentamt
European Patent Office
Office européen des brevets

(11) EP 1 487 778 B1

(12) **EUROPÄISCHE PATENTSCHRIFT**

(45) Veröffentlichungstag und Bekanntmachung des Hinweises auf die Patenterteilung:
**11.07.2012 Patentblatt 2012/28**

(21) Anmeldenummer: **03708253.4**

(22) Anmeldetag: **18.03.2003**

(51) Int Cl.:
***C07C 211/35*** (2006.01) ***C07C 211/37*** (2006.01)
***C07D 209/04*** (2006.01) ***C07D 333/46*** (2006.01)
***A61P 25/24*** (2006.01) ***A61P 25/28*** (2006.01)

(86) Internationale Anmeldenummer:
**PCT/EP2003/002812**

(87) Internationale Veröffentlichungsnummer:
**WO 2003/080557 (02.10.2003 Gazette 2003/40)**

(54) **SUBSTITUIERTE 4-AMINOCYCLOHEXANOLE**

SUBSTITUTED 4-AMINOCYCLOHEXANOLS

4-AMINOCYCLOHEXANOLS SUBSTITUES

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **23.03.2002 DE 10213051**

(43) Veröffentlichungstag der Anmeldung:
**22.12.2004 Patentblatt 2004/52**

(73) Patentinhaber: **Grünenthal GmbH**
**52078 Aachen (DE)**

(72) Erfinder:
- **SUNDERMANN, Bernd**
**52066 Aachen (DE)**
- **HENNIES, Hagen-Heinrich**
**52152 Simmerath (DE)**
- **ENGLBERGER, Werner**
**52223 Stolberg (DE)**
- **KÖGEL, Babette-Yvonne**
**52379 Langerwehe-Hamich (DE)**

(74) Vertreter: **Bülle, Jan**
**Kutzenberger & Wolff**
**Patentanwaltssozietät**
**Theodor-Heuss-Ring 23**
**50668 Köln (DE)**

(56) Entgegenhaltungen:
**WO-A-02/30891** **WO-A-96/14307**
**US-A- 4 366 172** **US-A- 4 446 065**

- **J. VIGNON ET AL.: "Biochemical properties of the brain phenylcyclidine receptor" EUROPEAN JOURNAL OF PHARMACOLOGY, Bd. 81, Nr. 4, 1982, Seiten 531-542, XP009012275**
- **A. KALIR ET AL.: "1-Phenylcycloalkylamine derivatives " ISRAEL JOURNAL OF CHEMISTRY, Bd. 13, Nr. 2, 1975, Seiten 125-136, XP009012270**
- **BERTORELLI R ET AL: "Nociceptin/orphanin FQ and its receptor: a potential target for drug discovery" TRENDS IN PHARMACOLOGICAL SCIENCES, ELSEVIER TRENDS JOURNAL, CAMBRIDGE, GB, Bd. 21, Nr. 7, 1. Juli 2000 (2000-07-01), Seiten 233-234, XP004209778 ISSN: 0165-6147**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft substituierte 4-Aminocyclohexanole, Verfahren zu deren Herstellung, Arzneimittel enthaltend diese Verbindungen und die Verwendung von substituierten 4-Aminocyclohexanolen zur Herstellung von Arzneimitteln zur Behandlung diverser Indikationen, insbesondere von Schmerz.

Das Heptadekapeptid Nociceptin ist ein endogener Ligand des ORL1 (Opioid-Receptor-Like)-Rezeptors (Meunier et al., Nature 377, 1995, S. 532-535). der zu der Familie der Opioid Rezeptoren gehört und in vielen Regionen des Gehirns und des Rückenmarks zu finden ist (Mollereau et al., FEBS Letters, 341, 1994, S. 33-38, Darland et al., Trends in Neurosciences, 21. 1998, S. 215-221). Das Peptid ist durch eine hohe Affinität, mit einem $K_d$-Wert von annähernd 56 pM (Ardati et al., Mol. Pharmacol. 51, S. 816-824), und durch eine hohe Selektivität für den ORL1-Rezeptor gekennzeichnet. Der ORL1-Rezeptor ist homolog zu den $\mu$, $\kappa$ und $\delta$ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/O}$-Proteinen zu einer Inhibierung der Adenylatcyclase (Meunier et al., Nature 377, 1995, S. 532-535). Auch auf der zellulären Ebene sind funktionelle Ähnlichkeiten der $\mu$. $\kappa$ und $\delta$ Opioid-Rezeptoren mit dem ORL1-Rezeptor in Bezug auf die Aktivierung des Kalium-Kanals (Matthes et al., Mol. Pharmacol. 50, 1996, S. 447-459; Vaughan et al., Br. J. Pharmacol. 117, 1996, S, 1609-1611) und der Inhibierung der L-, N- und P/Q-Typ-Kalzium-Kanäle vorhanden (Conner et al., Br. J. Pharmacol. 118, 1996, S. 205-207; Knoflach et al., J. Neuroscience 16, 1996, S. 6657-6664).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794; Hara et al,. Br. J. Pharmacol. 121, 1997, S. 401-408). Diese Befunde können als Hemmung der stressinduzierten Analgesie erklärt werden (Mogil et al., Neurosci. Letters 214, 1996, S131-134; sowie Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin hemmt die Aktivität Kainat- oder Glutamat-stimulierter Hinterwurzeiganglienneuronen (Shu et al., Neuropeptides, 32, 1998, 567-571) oder Glutamat-stimulierter Rückenmarksneuronen (Faber et al., Br. J. Pharmacol., 119, 1996, S. 189-190); es wirkt antinociceptiv im Tail Flick-Test in der Maus (King et al., Neurosci. Lett., 223, 1997, 113-116), im Flexor-Reflex-Modell in der Ratte (Xu et al., NeuroReport, 7, 1996, 2092-2094) und im Formalin-Tost an der Ratte (Yamamoto et al., Neuroscience, 81, 1997, S. 249-254). In Modellen für neuropathische Schmerzen konnte ebenfalls eine antinociceptive Wirkung von Nociceptin nachgewiesen werden (Yamamoto und Nozaki-Taguchi, Anesthesiology, 87, 1997), die insofern besonders interessant ist, als das die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Sandin et al., Eur. J. Neurosci., 9, 1997, S. 194-197; Manabe et al., Nature, 394, 1997, S. 577-581), Hörvermögen (Nishi et al., EMBO J., 16, 1997, S. 1858-1864), Nahrungsaufnahme (Pomonis et al., NeuroReport, 8, 1996, S. 369-371), Regulation des Blutdruckes (Gumusel et al., Life Sci., 60, 1997, S. 141-145; Campion und Kadowitz, Biochem. Biophys. Res. Comm., 234, 1997, S. 309-312), Epilepsie (Gutiérrez et al, Abstract 536.18, Society for Neuroscience, Vol 24, 28th Ann. Meeting, Los Angeles. November 7.-12, 1998) und Diurese (Kapista et al., Life Sciences, 60, 1997, PL 15-21). In einem Übersichtsartikel von Calo et al. (Br.J. Pharmacol., 129, 200D, 1261 -1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf $\mu$-Agonisten wie Morphin, Behandlung von Entzugserscheinungen, Reduzierung des Suchtpotentials von Morphinen, Anxiolyse, Modulation der Bewegungsaktivität, Gedächtnis-Störungen, Epilepsie: Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, aterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren.

Substituierte 4-Aminocyclohexanole, in denen die Aminofunktion Teil eines heterocyclischen Ringes ist oder eine primäre Aminofunktion darstellt, sind bereits bekannt (A Kalir et al., Israel Journal of chemistry, 13(2), 125-136 (1975); J. Vignon et al., European journal of pharmacology, 81(4), 531-542 (1982)).

Aus US 4,366,172 und WO 96/14307 sind außerdem 4-Aryl-substituierte 4-Aminocyclohexanole bekannt.

Aufgabe der vorliegenden Erfindung war es, Wirkstoffe zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankeiten bzw, zum Einsatz in den dort genannten Indikationen geeignet sind.

Gegenstand der Erfindung sind daher substituierte 4-Aminocyclohexanole gemäß der allgemeinen Formel I,

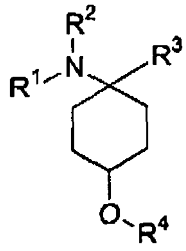

I

, worin

$R^1$ ausgewählt ist aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,

$R^2$ ausgewählt ist aus H; $C_{1-4}$-Alkyl, gesättigt oder ungesättigt, verzweigt oder unverzweigt, einfach oder mehrfach substituiert oder unsubstituiert

$R^3$ ausgewählt ist aus $C_{1-8}$-Alkyl, gesättigt oder ungesättigt, verzweigt oder unverzweigt, einfach oder mehrfach substituiert oder unsubstituiert; $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert; über eine $C_{1-3}$-Alkyl Brücke gebundenem Aryl oder $C_{3-8}$-Cycloalkyl, jeweils einfach oder mehrfach substituiert oder unsubstituiert oder über eine $C_{1-3}$-Alkyl Brücke gebundenem Heterocyclyl, gesättigt oder ungesättigt, einfach oder mehrfach substituiert oder unsubstituiert;

$R^4$ ausgewählt ist aus $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert; $-CHR^6R^7$, $-CHR^6-CH_2R^7$, $-CHR^6-CH_2-CH_2R^7$, $-CHR^6-CH_2-CH_2-CH_2R^7$; oder $-R^8-L-R^9$

mit $R^6$ ausgewählt aus

H; $C_{1-8}$-Alkyl, gesättigt oder ungesättigt, verzweigt oder unverzweigt, einfach oder mehrfach substituiert oder unsubstituiert; $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert; oder $C(O)O-R^{10}$;
mit $R^{10}$ ausgewählt aus

$C_{1-7}$-Alkyl, gesättigt oder ungesättigt, verzweigt oder unverzweigt, einfach oder mehrfach substituiert oder unsubstituiert; $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert;

mit $R^7$ ausgewählt aus

H; $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert,

mit $R^8$ ausgewählt aus

$C_{3-8}$-Cycloalkyl, einfach oder mehrfach substituiert oder unsubstituiert; Aryl, einfach oder mehrfach substituiert

oder unsubstituiert; Heterocyclyl, gesättigt oder ungesättigt, einfach oder mehrfach substituiert oder unsubstituiert,

mit L ausgewählt aus

-C(O)-NH-, -NH-C(O)-, -C(O)-O-, -O-C(O)-, -O-, -S- oder $-S(O)_2-$

mit $R^9$ ausgewählt aus

H; $C_{1-8}$-Alkyl, gesättigt oder ungesättigt, verzweigt oder unverzweigt, einfach oder mehrfach substituiert oder unsubstituiert; $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert;

wobei der Begriff "substituiert" im Zusammenhang mit Alkyl und Cycloalkyl die Substitution eines Wasserstoffrestes durch F, Cl, Br, I, $NH_2$, SH oder OH bedeutet, wobei unter "mehrfach substituiert" Resten zu verstehen ist, daß die Substitution sowohl an verschiedenen als auch an gleichen Atomen mehrfach mit den gleichen oder verschiedenen Substituenten erfolgt;

wobei "substituiert" im Zusammenhang mit Aryl und Heteroaryl die Substitution des Aryls oder Heteroaryls mit $R^{23}$, $OR^{23}$, einem Halogen, einem $CF_3$, einem CN, einem $NO_2$, einem $NR^{24}R^{25}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen bedeutet;

wobei

der Rest $R^{23}$ für H, einen $C_{1-10}$-Alkyl-, einen Aryl- oder Heteroaryl-oder für einen über eine $C_{1-3}$-Alkylen-Gruppe gebundenen Aryl-oder Heteroaryl-Rest steht, wobei diese Aryl und Heteroarylreste nicht selbst mit Aryl- oder Heteroaryl-Resten substituiert sein dürfen,

die Reste $R^{24}$ und $R^{25}$, gleich oder verschieden, für H, einen $C_{1-10}$-Alkyl-, einen Aryl-, einen Heteroaryl- oder einen über eine $C_{1-3}$-Alkylen-Gruppe gebundenen Aryl- oder Heteroaryl-Rest stehen, wobei diese Aryl und Heteroarylreste nicht selbst mit Aryl- oder Heteroaryl-Resten substituiert sein dürfen,

oder die Reste $R^{24}$ und $R^{25}$ zusammen $CH_2CH_2OCH_2CH_2$, $CH_2CH_2NR^{26}CH_2CH_2$ oder $(CH_2)_{3-6}$ bedeuten, und

der Rest $R^{26}$ für H, einen $C_{1-10}$-Alkyl-, einen Aryl-, oder Heteroaryl-Rest oder für einen über eine $C_{1-3}$-Alkylen-Gruppe gebundenen Aryl- oder Heteroaryl-Rest steht, wobei diese Aryl und Heteroarylreste nicht selbst mit Aryl- oder Heteroaryl-Resten substituiert sein dürfen;

in Form ihrer Racemate, ihrer reinen Enantiomere oder Diastereomere, Mischungen der Enantiomere oder Diastereomere in einem beliebigen Mischungsverhältnis; in dargestellter Form oder in Form ihrer physiologisch verträglichen Salze, oder in Form ihrer Solvate, insbesondere der Hydrate.

Alle diese erfindungsgemäßen Verbindungen bzw. Verbindungsgruppen zeigen hervorragende Bindung an den ORL1-Rezeptor und zeigen analgetische Wirksamkeit.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für $C_{1-}$, C2- oder C3-Alkyl, $C_{1-4}$-Alkyl für C1-, C2-, C3- oder C4-Alkyl, $C_{1-5}$-Alkyl für $C_{1-}$, C2-, C3-, C4-oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-. C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3-oder C4-Cycloalkyl, $C_{3-5}$-Cycloalkyl für C3-, C4- oder C5-Cycloalkyl, $C_{3-6}$-Cycloalkyl für C3-, C4-, C5- oder $C_6$-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyl ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - wenn nicht ausdrücklich anders definiert - unter dem Begriff substituiert im Sinne dieser Erfindung die Substitution eines Wasserstoffrestes durch F, Cl, Br, I, $NH_2$, SH oder OH, wobei unter "mehrfach substituiert" Resten zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von -CH(OH)-CH=CH-$CHCl_2$. Besonders bevorzugte Substituenten sind hier F, Cl und OH.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$ - $CH_2-CH_2-$, $-CH_2-CH_2-CH_2-$ und-$CH_2-CH_2-CH_2-CH_2-$zu verstehen, etc.

Unter einem Aryl-Rest werden Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der

Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo-1,2,5 thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert - wenn nicht ausdrücklich anders definiert - die Substitution des Aryls oder Heteroaryls mit $R^{23}$, $OR^{23}$ einem Halogen, vorzugsweise F und/oder Cl, einem $CF_3$, einem CN, einem $NO_2$, einem $NR^{24}R^{25}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{23}$ für H, einen $C_{1-10}$-Alkyl-, vorzugsweise einen $C_{1-6}$-Alkyl-, einen Aryl- oder Heteroaryl- oder für einen über eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste $R^{24}$ und $R^{25}$, gleich oder verschieden, für H, einen $C_{1-10}$-Alkyl-, vorzugsweise einen $C_{1-6}$-Alkyl-, einen Aryl-, einen Heteroaryl- oder einen über eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste $R^{24}$ und $R^{25}$ bedeuten zusammen $CH_2CH_2OCH_2CH_2$, $CH_2CH_2NR^{26}CH_2CH^2$ oder $(CH_2)_{3-6}$, und der Rest $R^{26}$ für H, einen $C_{1-10}$-Alkyl-, vorzugsweise einen $C_{1-6}$-Alkyl-, einen Aryl-, oder Heteroaryl- Rest oder für einen über eine $C_{1-3}$-Alkylen-Gruppe gebundenen Aryl- oder Heteroaryl-Rest, wobei diese Aryl und Heteroarylreste nicht selbst mit Aryl-oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure. Oxalsäure, Bernsteinsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt ist das Hydrochlorid-Salz.

Besonders bevorzugt sind substituierte 4-Aminocyclohexanole, bei denen

$R^1$ und $R^2$ Methyl bedeuten.

Besonders bevorzugt sind substituierte 4-Aminocyclohexanole, bei denen

$R^3$ ausgewählt ist aus $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert; über eine $C_{1-3}$-Alkyl Brücke gebundenem Aryl oder $C_{3-8}$-Cycloalkyl, jeweils einfach oder mehrfach substituiert oder unsubstituiert oder über eine $C_{1-3}$-Alkyl Brücke gebundenem Heterocyclyl, gesättigt oder ungesättigt, jeweils einfach oder mehrfach substituiert oder unsubstituiert;

vorzugsweise

$R^3$ ausgewählt ist aus $C_{5-6}$-Cycloalkyl, unsubstituiert oder einfach oder mehrfach substituiert; Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, oder über eine gesättigte, unverzweigte $C_{1-2}$-Alkyl-Gruppe gebundenem $C_{5-6}$-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert;

insbesondere

$R^3$ ausgewählt ist aus Phenyl, Pyridyl, Furyl oder Thiophenyl, jeweils unsubstituiert oder einfach oder mehrfach substituiert; oder über eine gesättigte, unverzweigte $C_{1-2}$-Alkyl-Gruppe gebundenem Phenyl, Pyridyl, Furyl oder Thiophenyl, jeweils unsubstituiert oder einfach oder mehrfach substituiert.

Besonders bevorzugt sind substituierte 4-Aminocyclohexanole, bei denen

$R^4$ ausgewählt ist aus $C_{3-8}$-Cycloalkyl, Aryl oder Heteroaryl, jeweils unsubstituiert oder einfach oder mehrfach substituiert; oder $-R^8-L-R^9$

vorzugsweise

$R^4$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2.5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert; oder $-R^8-L-R^9$

insbesondere

$R^4$ ausgewählt ist aus Cyclopentyl, Cyclohexyl, Cycloheptyl, Cyclooctyl, Anthracenyl, Indolyl, Naphthyl, Benzothiazolyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert: oder $-R^8-L-R^9$.

Dabei ist es für beide vorangehend beschriebenen bevorzugten Ausführungsformen vorteilhaft, wenn

$R^8$ ausgewählt ist aus

Indolyl, Naphthyl, Benzofuranyl, Benzothiophenyl, Indanyl, Benzodioxanyl, Benzodioxolanyl, Acenaphthyl, Carbazolyl, Phenyl. Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl,

jeweils unsubstituiert oder einfach oder mehrfach substituiert,

L ausgewählt aus

-C(O)-NH-, NH-C(O)-. -C(O)-O-, -O-C(O)-, -O-, -S- oder $-S(O)_2-$,

und/oder $R^9$ ausgewählt ist aus

Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,

vorzugsweise

$R^8$ ausgewählt ist aus

Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert,

L ausgewählt aus

-C(O)-NH-, -NH-C(O)-, -C(O)-O-, -O-C(O)- oder $-S(O)_2-$,

und/oder $R^9$ ausgewählt ist aus

Indolyl, Benzothiophenyl, Phenyl, Thiophenyl, Furyl, Pyridyl, Pyrrolyl, Pyrazinyl oder Pyrimidyl, jeweils unsubstituiert oder einfach oder mehrfach substituiert

insbesondere

$R^8$ ausgewählt ist aus

Indolyl, unubstituiert.

L ausgewählt aus

$-S(O)_2-$

und $R^9$ ausgewählt ist aus

Phenyl unsubstituiert.

Besonders bevorzugt sind substituierte 4-Aminocyclohexanole, bei denen

$R^4$ ausgewählt ist aus $-CHR^6R^7$, $-CHR^6$- $CH_2R^7$, $-CHR^6-CH_2-CH_2R^7$, $-CHR^6-CH_2-CH_2-CH_2R^7$,

vorzugsweise

$R^4$ ausgewählt ist aus $-CHR^6R^7$, $-CHR^6$- $CH_2R^7$ oder $-CHR^6-CH_2-CH_2R^7$,

insbesondere

$R^4$ ausgewählt ist aus $-CHR^6R^7$ oder $-CHR^6$- $CH_2R^7$.

Dabei ist es für beide vorangehend beschriebenen bevorzugten Ausführungsformen vorteilhaft, wenn

$R^6$ ausgewählt ist aus

H, $C_{1-4}$-Alkyl, gesättigt oder ungesättigt, verzweigt oder unverzweigt, einfach oder mehrfach substituiert oder unsubstituiert; oder C(O)O $R^{10}$

mit $R^{10}$ ausgewählt aus $C_{1-4}$-Alkyl, gesättigt oder ungesättigt, verzweigt oder unverzweigt, einfach oder mehrfach substituiert oder unsubstituiert;

vorzugsweise

H, $C_{1-4}$-Alkyl, gesättigt oder ungesättigt, verzweigt oder unverzweigt, einfach oder mehrfach substituiert oder unsubstituiert;

insbesondere

H, $CH_3$ und $C_2H_5$.

Dabei ist es für die drei vorangehend beschriebenen bevorzugten Ausführungsformen vorteilhaft, wenn

$R^7$ ausgewählt ist aus $C_{3-8}$-Cycloalkyl, Aryl oder Heteroaryl, jeweils unsubstituiert oder einfach oder mehrfach substituiert;

vorzugsweise

$R^7$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazoly) oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;

insbesondere

$R^7$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Besonders bevorzugt ist es, wenn die erfindungsgemäßen substituierten 4-Aminocyclohexanole ausgewählt sind aus der folgenden Gruppe:

- trans-(4-Benzyloxy-1-phenylcyclohexyl)dimethylamin
- cis-(4-Benzyloxy-1-phenylcyclohexyl)dimethylamin
- trans-(1-Benzyl-4-benzyloxycyclohexyl)dimethylamin
- cis-(1-Benzyl-4-benzyloxycyclohexyl)dimethylamin
- trans-[4-Benzyloxy-1-(2-methylbenzyl)cyclohexyl]dimethylamin
- cis-[4-Benzyloxy-1-(2-methylbenzyl)cyclohexyl]dimethylamin
- cis-[4-(2-Fluorbenzyloxy)-1-phenylcyclohexyl]dimethylamin
- cis-[1-Benzyl-4-(3-fluorbenzyloxy)cyclohexyl]dimethylamin
- cis-[1-Benzyl-4-(2-fluorbenzyloxy)cyclohexyl]dimethylamin
- cis-[1-Benzyl-4-(4-fluorbenzyloxy)cyclohexyl]dimethylamin
- trans-[4-(2-Fluorbenzyloxy)-1-phenylcyclohexyl]dimethylamin
- trans-[4-(3-Fluorbenzyloxy)-phenylcyclohexyl]dimethylamin
- trans-[4-(4-Fluorbenzyloxy)-1-phenylcyclohexyl]dimethylamin
- trans-[4-(4-Fluorbenzyloxy)-phenethylcyclohexyl]dimethylamin
- trans-[4-(3-Fluor-benzyloxy)-1-phenethylcyclohexyl]dimethylamin

in Form ihrer Racemate, ihrer reinen Enantiomeren oder Diastereomeren, oder in Form von Mischungen der

Enantiomeren oder Diastereomeren, in einem beliebigen Mischungsverhältnis; in dargestellter Form oder in Form ihrer physiologisch verträglichen Salze, oder in Form ihrer Solvate, insbesondere der Hydrate.

Die erfindungsgemäßen Substanzen sind toxikologisch unbedenklich, so daß sie sich als pharmazeutischer Wirkstoff in Arzneimitteln eignen. Ein weiterer Gegenstand sind Arzneimittel enthaltend mindestens ein erfindungsgemäßes 4-Aminocyclohexanol in Form seines Racemats, seiner reinen Enantiomeren oder Diastereomeren, oder in Form von Mischungen der Enantiomeren oder Diastereomeren, in einem beliebigen Mischungsverhältnis; in dargestellter Form oder in Form seiner physiologisch verträglichen Salze, oder in Form seiner Solvate, insbesondere der Hydrate, sowie gegebenenfalls geeignete Zusatz- und/oder Hilfsstoffe und/oder gegebenenfalls weitere Wirkstoffe.

Dabei ist es bevorzugt, wenn das Arzneimittel neben wenigstens einem erfindungsgemäßen substituierten 4-Aminocyclohexanol noch ein Opioid, vorzugsweise ein starkes Opioid, insbesondere Morphin, oder ein Anesthetikum, vorzugsweise Hexobarbital oder Halothan, enthält.

Die erfindungsgemäßen Arzneimittel enthalten neben mindestens eines erfindungsgemäßen 4-Aminocyclohexanols, gegebenenfalls geeignete Zusatz-und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße 4-Aminocyclohexanole in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen 4-Aminocyclohexanole verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines efindungsgemäßen 4-Aminocyclohexanols appliziert.

Ein weiterer Gegenstand der Erfindung ist die Verwendung eines erfindungsgemäßen substituierten 4-Aminocyclohexanols in Form seiner Racemate, seiner reinen Enantiomer oder Diastereomere, oder in Form von Mischungen der Enantiomeren oder Diastereomeren, in einem beliebigen Mischungsverhältnis; in dargestellter Form oder in Form seiner physiologisch verträglichen Salze, oder in Form seiner Solvate, insbesondere der Hydrate, zur Herstellung eines Arzneimittels zur Behandlung von Schmerz, insbesondere von akutem neuropathischem oder chronischem Schmerz.

Ebenfalls ein weiterer Gegenstand der Erfindung ist die Verwendung eines erfindungsgemäßen substituierten 4-Aminocyclohexanols in Form seiner Racemate, seiner reinen Enantiomeren oder Diastereomeren, oder in Form von Mischungen der Enantiomeren oder Diastereomeren in einem beliebigen Mischungsverhältnis; in dargestellter Form oder in Form seiner physiologisch verträglichen Salze, oder in Form seiner Solvate, insbesondere der Hydrate, zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese und/oder Anxiolyse.

Ein weiterer Gegenstand der Erfindung ist ein Verfahren zur Herstellung der erfindungsgemäßen substituierten 4-Aminocyclohexanole wie in der folgenden Beschreibung und Beispielen ausgeführt.

Ein weiterer Gegenstand der Erfindung ist ein Verfahren zur Herstellung eines erfindungsgemäßen substituierten 4-Aminocyclohexanols mit folgenden Schritten:

a. ein mit den Gruppen $S^1$ und $S^2$ geschütztes Cyclohexan-1,4-dion gemäß Formel IV wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel V umgesetzt;

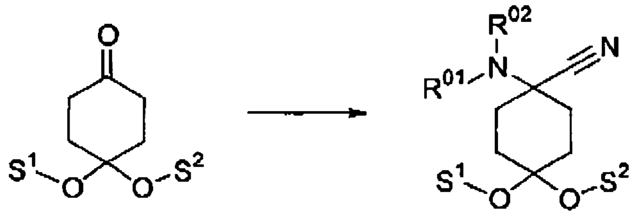

IV V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ = mit einer Schutzgruppe geschütztern H, mindestens einmal eine Schutzgrupe abgespalten und gegebenfalls acyliert, alkyliert oder sulfoniert und/oder bei einer Verbindungen mit $R^{01}$ und/oder $R^{02}$ = H mindestens einmal eine Schutzgruppe eingeführt und gegebenfalls acyliert, alkyliert oder sulfoniert,

b. das Aminonitril gemäß Formel V wird mit metallorganischen Reagenzien, bevorzugt Grignard- oder Organolithiumreagenzien, der Formel Metall-$R^3$ umgesetzt, so daß eine Verbindung gemäß Formel VI entsteht;

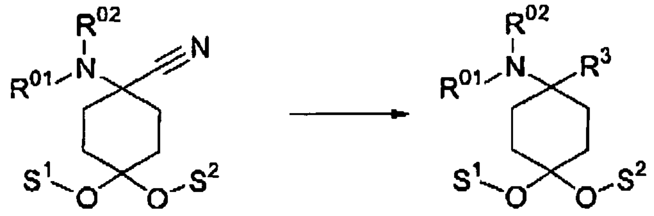

V VI

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ = mit einer Schutzgruppe geschütztern H, mindestens einmal eine Schutzgrupe abgespalten und gegebenfalls acyliert, alkyliert oder sulfoniert und/oder bei einer Verbindungen mit $R^{01}$ und/oder $R^{02}$ = H mindestens einmal eine Schutzgruppe eingeführt und gegebenfalls acyliert, alkyliert oder sulfoniert,

c. an der Verbindung gemäß Formel VI werden die Schutzgruppen $S^1$ und $S^2$ abgespalten, so daß ein 4-substituiertes 4-Aminocyclohexanonderivat gemäß Formel VII entsteht:

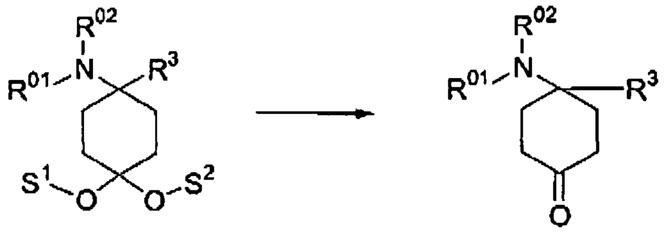

VI VII

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ = mit einer Schutzgruppe geschütztern H, mindestens einmal eine Schutzgrupe abgespalten und gegebenfalls acyliert, alkyliert oder sulfoniert und/oder bei einer Verbindungen mit $R^{01}$ und/oder $R^{02}$ = H mindestens einmal eine Schutzgruppe eingeführt und gegebenfalls acyliert, alkyliert oder sulfoniert,

d. das 4-substituierte 4-Aminocyclohexanonderivat gemäß Formel VII wird mit einem Reduktionsmittel wie beispielsweise Natriumborhydrid, Natriumcyanoborhydrid, Natriumtriacetoxyborhydrid, Lithiumaluminiumhydrid, Diisobutylaluminiumhydrid, einem komplexen Analogon dieser Verbindungen bei Temperaturen zwischen -70 °C und +110 °C, oder edelmetallkatalysiert mit Wasserstoff zu einem 4-Aminocyclohexanolderivat gemäß Formel VIII umgesetzt;

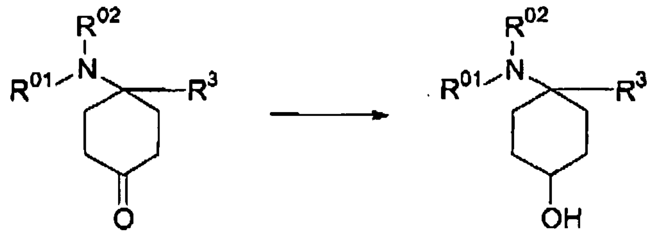

VII VIII

e. das 4-substituierte 4-Aminocyclohexanolderivat gemäß Formel VIII wird anschließend in Gegenwart einer anorganischen, metallorganischen oder organischen Base mit einem Alkyl-, Acyl- oder Arylbromid, -chlorid, -iodid, -triflat oder mit einem mit einer anderen Abgangsgruppe X versehenen Alkan, Alkylsäure oder Aromaten $R^4X$ zu einer Verbindung gemäß Formel I umgesetzt,

wobei $R^1$, $R^2$, $R^3$ und $R^4$ die für die substituierten 4-Aminocyclohexanole gemäß der allgemeinen Formel I angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert.

Ein weiterer Gegenstand der Erfindung ist ein alternatives Verfahren zur Herstellung der erfindungsgemäßen 4-Aminocyclohexanole mit folgenden Schritten:

a. ein mit den Gruppen $S^1$ und $S^2$ geschütztes Cyclohexan-1,4-dion gemäß Formel IV mit einem Reduktionsmittel wie beispielsweise Natriumborhydrid, Natriumcyanoborhydrid, Natriumtriacetoxyborhydrid, Lithiumaluminiumhydrid, Diisobutylaluminiumhydrid, einem komplexen Analogon dieser Verbindungen bei Temperaturen zwischen -70°C und +110 °C, oder edelmetallkatalysiert mit Wasserstoff zu einem geschützten 4-Hydroxycyclohexanonderivat gemäß Formel XIII umgesetzt;

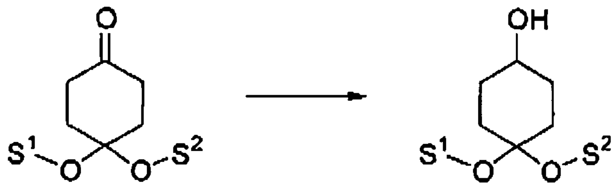

IV XIII

b. das anschließend in Gegenwart einer anorganischen, metallorganischen oder organischen Base mit einem Alkyl- oder Arylbromid, -chlorid, -iodid, -triflat oder mit einem mit einer anderen Abgangsgruppe X versehenen Alkan oder Aromaten $R^4X$ zu einer Verbindung gemäß Forme XIV umgesetzt wird;

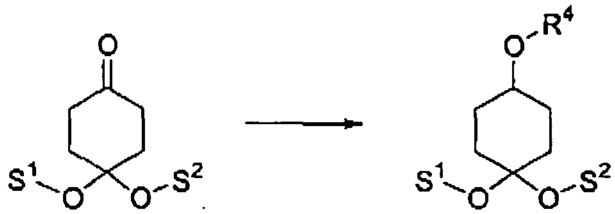

XIII XIV

c. an der Verbindung gemäß Formel XIV werden die Schutzgruppen $S^1$ und $S^2$ abgespalten, so daß eine Verbindung

gemäß Formel XV entsteht;

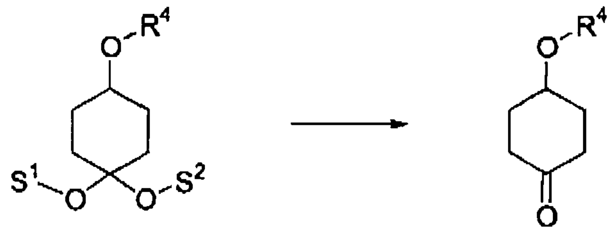

XIV XV

d. die Verbindung Formel XV wird in Gegenwart einer Verbindung der Formel $HNR^{01}R^{02}$ mit Cyanid, vorzugsweise Kaliumcyanid, zu einem α-Aminonitrilderivat der Formel XVI umgesetzt,

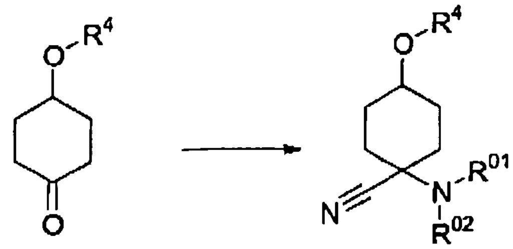

XV XVI

e. das α-Aminonitrilderivat der Formel XVI wird mit metallorganischen Reagenzien, bevorzugt Grignard- oder Organolithiumreagenzien, der Formel Metall-$R^3$ zu einer Verbindung gemäß Formel umgesetzt,

wobei $R^1$, $R^2$, $R^3$ und $R^4$ die für die substituierten 4-Aminocyclohexanole gemäß der allgemeinen formel I angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert.

Im folgenden wird die Erfindung weiter durch Beispiele erläutert, ohne sie darauf zu beschränken.

## Beispiele

Die folgenden Beispiele dienen zur näheren Erläuterung der Erfindung, schränken aber den allgemeinen Erfindungsgedanken nicht ein,

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." bedeutet Schmelzpunkt bzw. Schmelzbereich, "RT" bedeutet Raumtemperatur, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/ Volumen angegeben.

Beispiel 1 & 2: (4-Benzyloxy-1-phenyl-cyclohexyl)dimethylamin Hydrochlorid, unpolareres und polareres Diastereomer

350 g 1,4-Dioxa-spiro[4.5]decan-8-on wurden in 2000 ml Ethanol suspendiert und bei Eisbadkühlung 28,1 g

Natriumborhydrid portionsweise zugegeben. Nach Rühren bei RT über Nacht wurden unter Rühren zunächst 750 ml Phosphatpuffer (pH 7, Merck-Darmstadt), dann 1000 ml Diethylether zugegeben, ausgefallene Feststoffe abfiltriert und mit Diethylether nachgewaschen. Das Filtrat wurde über Natriumsulfat getrocknet, filtriert und eingeengt. Es wurden 345 g 1,4-Dioxa-spiro[4.5]decan-8-ol als weißer Festoff erhalten.

225 g 1,4-Dioxa-spiro[4.5]decan-8-ol wurden in 1100 ml Dimethylformamid gelöst und unter Rühren portionsweise 198 g Kalium tert.-butylat zugegeben. Nach einer Stunden wurden innerhalb 30 Minuten 223 g Benzylchlorid zugetropft und über Nacht nachgerührt. Der Ansatz wurde auf 1500 ml Wasser/Eis-Gemisch gegeben und mit Ethylacetat extrahiert. Die vereinigten Extrakte wurden mit gesättigter Natriumchloridlösung gewaschen, über Natriumsulfat getrocknet, filtriert und eingeengt. Das Rohprodukt wurde im Hochvakuum fraktioniert destilliert. Bei 150 °C und einem Druck um 0,1 mbar wurden 294 g 8-Benzyloxy-1,4-dioxaspiro[4.5]decan erhalten.

294 g 8-Benzyloxy-1,4-dioxaspiro[4.5]decan wurden in 1400 ml Diisopropylether gelöst und 580 ml viermolare Salzsäure zugegeben. Nach 20 Stunden Rühren bei RT wurden 300 ml Wasser und 180 ml gesättigte Natriumchloridlösung zugegeben und weitere vier Stunden gerührt. Die Phasen wurden getrennt, die wäßrige Phase mit festem Natriumhydrogencarbonat neutralisiert und wiederholt mit Diisopropylether extrahiert. Die vereinigten organischen Phasen wurden über Natriumsulfat getrocknet, filtriert und eingeengt. Durch Destillation im Hochvakuum wurden 231 g 4-Benzyloxycyclohexanon erhalten

Ein Gemisch aus 130 ml 7,9 molarer wäßriger Dimethylaminlösung, 16 ml Methanol, 20,0 g 4-Benzyloxycyclohexanon, 23,2 g Dimethylamin Hydrochlorid und 15,3 g Kaliumcyanid wurde 65 Stunden bei RT gerührt, viermal mit je 120 ml Diethylether extrahiert, die vereinigten Extrakte eingeengt, 100 ml Dichlormethan zum Rückstand hinzugefügt, die Phasen getrennt, die organische Phasen wurden über Natriumsulfat getrocknet, filtriert und eingeengt. Es wurden 24,5 g 4-Benzyloxy-1-dimethylamino-cyclohexancarbonitril als langsam erstarrendes gelbliches Öl erhalten.

5,00 g 4-Benzyloxy-1-dimethylaminocyclohexancarbonitril wurden in 50 ml Tetrahydrofuran gelöst und bei Eisbadkühlung 19,4 ml zweimolarer Phenylmagnesiumchloridlösung in Tetrahydrofuran unter Stickstoffatmosphäre zugetropft. Nach Rühren über Nacht unter Erwärmung auf RT wurde erneut im Eisbad gekühlt und 25 ml kalter Ammoniumchloridlösung (20 massenprozentig) zugegeben. Die Phasen wurden getrennt, zweimal mit je 80 ml Diethylether extrahiert, die vereinigten organischen Phasen dreimal mit je 60 ml Salzsäure (5 massenprozentig) extrahiert. die vereinigten sauren Extrakte mit wäßriger Ammoniaklösung (25 massenprozentig) schwach alkalisch gestellt (pH 8-9), dreimal mit je 80 ml Diethylether extrahiert, die vereinigten organischen Extrakte über Natriumsulfat getrocknet, filtriert und eingeengt. Das erhaltene gelbe Öl (4,15 g) wurde mit Diethylether/Hexan (V:V = 1:1) an Kieselgel getrennt. Es wurden 1,83 g des unpolareren und 0,38 g des polareren Diastereomers von (4-Benzyloxy-1-phenyl-cyclohexyl)dimethylamin als gelber Feststoff bzw. gelbes Harz erhalten. 1,48 g des unpolareren Diastereomers wurden in 11,8 ml 2-Butanon gelöst, 47 µl Wasser und 665 µl Chlortrimethylsilan hinzugegeben und über Nacht gerührt. Das ausgefallene Hydrochlorid wurde abfiltriert, mit Diethylether gewaschen und im Hochvakuum getrocknet. Es wurden 1,55 g des Hydrochlorids des unpolareren Diastereomers von (4-Benzyloxy-1-phenyl-cyclohexyl)dimethylamin (Beispiel 1) als weißer Feststoff erhalten. Analog wurden aus 379 mg des polareren Diastereomers 300 mg des korrespondierenden Hydrochlorid (Beispiel 2) erhalten.

Beispiel 3: (1-Benzyl-4-benzyloxy-cyclohexyl)dimethylamin Hydrochlorid

4,00 g 4-Benzyloxy-1-dimethylaminocyclohexancarbonitril wurden in 40 ml Tetrahydrofuran gelöst und bei Eisbadkühlung 10,8 ml zweimolarer Benzylmagnesiumchloridlösung in Tetrahydrofuran unter Stickstoffatmosphäre zugetropft. Nach Rühren über Nacht unter Erwärmung auf RT wurde erneut im Eisbad gekühlt und 20 ml kalter Ammoniumchloridlösung (20 massenprozentig) zugegeben. Die Phasen wurden getrennt, zweimal mit je 60 ml Diethylether extrahiert, die vereinigten organischen Phasen dreimal mit je 50 ml Salzsäure (5 massenprozentig) extrahiert, die vereinigten sauren Extrakte mit wäßriger Ammoniaklösung (25 massenprozentig) schwach alkalisch gestellt (pH 8-9), dreimal mit je 60 ml Diethylether extrahiert, die vereinigten organischen Extrakte über Natriumsulfat getrocknet, filtriert und eingeengt. Der erhaltene gelbe Feststoff (3,85 g) wurde mit Diethylether/Hexan (V:V = 1:1) an Kieselgel getrennt. Es wurden 2,45 g (1-Benzyl-4-benzyloxycyclo-hexyl)dimethylamin als weißer Feststoff erhalten, die, wie für Beispiel 1 beschrieben, mit Chlortrimethylsilan und Wasser in 2-Butanon in 2,03 g des korrespondierenden Hydrochlorids überführt wurden (weißer Feststoff).

Beispiel 4: [4-(2-Fluor-benzyloxy)-1-phenyl-cyclohexyl]dimethylamin

200 g 1,4-Dioxa-spiro[4,5]decan-8-on wurden mit 200 ml Methanol, 1680 ml wäßriger Dimethylaminlösung (40 massenprozentig), 303 g Dimethylamin Hydrochlorid und 200 g Kaliumcyanid versetzt und für ca. 65 Stunden gerührt. Die erhaltene weiße Suspension wurde viermal mit je 800 ml Ether extrahiert, die vereinigten Extrakte eingeengt, der Rückstand in ca. 500 ml Dichlormethan aufgenommen und die Phasen getrennt. Die Dichlormethanphase wurde Ober Natriumsulfat getrocknet, filtriert und eingeengt. Es wurden 265 g 8-Dimethylamino-1,4-dioxa-spiro[4.5]decan-8-carbonitril als weißer Feststoff erhalten.

50,0 g 8-Dimethylamino-1,4-dioxa-spiro[4.5]decan-8-carbonitril wurden in 400 ml Tetrahydrofuran p.a. gelöst, bei Eisbadkühlung unter Stickstoffatmosphäre 216 ml einer kommerziell erhältlichen zweimolaren Lösung von Phenylmagnesiumchlorid in Tetrahydrofuran zugetropft und Ober Nacht unter Erwärmung auf Raumtemperatur gerührt. Zur Aufarbeitung wurden unter Rühren bei Eisbadkühlung 200 ml eiskalter Ammoniumchloridlösung (20 m%) zugegeben und nach 30 Minuten die Phasen getrennt. Die wäßrige Phase wurde zweimal mit je 250 ml Ether extrahiert, die Extrakte mit der organischen Phase vereinigt, mit 200 ml Wasser gefolgt von 200 ml gesättigter Natriumchloridlösung gewaschen, über Natriumsulfat getrocknet, filtriert und eingeengt. Es wurden 60,0g Dimethyl-(8-phenyl-1,4-dioxa-spiro[4.5]dec-8-yl)-amin erhalten.

165 ml Salzsäure (32 m%) wurden mit 100 ml Wasser verdünnt, in diese ca. sechsmolare Salzsäure 60.0 g Dimethyl-(8-phenyl-1,4-dioxa-spiro[4.5]dec-8-yl)-amin gegeben und 24 Stunden gerührt. Das Reaktionsgemisch wurde dreimal mit je 50 ml Diethylether gewaschen, mit 100 ml Natronlauge (32 m%) alkalisch gestellt (pH > 10) und dreimal mit je 100 ml Dichlormethan extrahiert. Die Extrakte wurden vereinigt. über Natriumsulfat getrocknet, filtriert und eingeengt. Es wurden 36,1 g 4-Dimethylamino-4-phenylcyclohexanon erhalten.

3,35 g 4-Dimethylamino-4-phenylcyclohexanon wurden in 25 ml Isopropanol suspendiert, bei Eisbadkühlung 620 mg Natriumboranat zugegeben und unter Erwärmung auf RT über Nacht gerührt. Es wurden 6,5 ml Phospharpuffer (pH 7, Merck-Darmstadt) zugetropft und der Ansatz eingeengt. Der Rückstand wurde mit 10 ml Wasser und 20 ml Diethylether aufgenommen und mit Kaliumhydroxid alkalisch gestellt. Die Phasen wurden getrennt und dreimal mit je 15 ml Diethylether extrahiert. Die vereinigten organischen Phasen wurden über Natriumsulfat getrocknet, filtriert und eingeengt. Es wurden 2,75 g 4-Dimethylamino-4-phenylcyclo-hexanol erhalten.

2,75 g 4-Dimethylamino-4-phenylcyclohexanol wurden in 20 ml Dimethylformanid gelöst, 1,55 g Kalium tert.-butytat zugegeben und 45 Minuten gerührt, bevor 2,0 g 2-Fluorbenzylchlohd innerhalb 30 Minuten zugetropft wurden. Nach Rühren über Nacht wurde der Ansatz auf 25 ml Eiswasser gegeben und wiederholt mit Ethylacetat extrahiert. Die vereinigten Extarkte wurden mit gesättigter Natriumchloridlösung gewaschen, über Natriumsulfat getrocknet, filtriert und eingeengt. Das erhaltene Rohprodukt (3.41 g) wurde mit Methanol/Ethylacetat (V:V = 1;1) an Kieselgel getrennt. Es wurden 580 mg [4-(2-Fluor-benzyloxy)-1-phenylcyclohexyl]dimethylamin als weißer Feststoff erhalten, die, wie für Beispiel 1 beschrieben, mit Chlortrimethylsilan und Wasser in 2-Butanon in 370 mg des korrespondierenden Hydrochlorids überführt wurden.

Beispiel 5: [1-Benzyl-4-(3-fluor-benzyloxy)-cyclohexy]dimethylamin

50,0 g 8-Dimethylamino-1,4-dioxa-spiro[4.5]decan-8-carbonitril wurden in 400 ml Tetrahydrofuran p.a. gelöst, bei Eisbadkühlung unter Stickstoffatmosphäre 214 ml einer kommerziell erhältlichen zweimolaren Lösung von Benzylmagnesiumchlorid in Tetrahydrofuran zugetropft und über Nacht unter Erwärmung auf Raumtemperatur gerührt. Zur Aufarbeitung wurden unter Rühren bei Eisbadkühlung 200 ml eiskalter Ammoniumchloridlösung (20 massenprozentig) zugegeben und nach 30 Minuten die Phasen getrennt. Die wäßrige Phase wurde zweimal mit je 250 ml Ether extrahiert, die Extrakte mit der organischen Phase vereinigt, mit 200 ml Wasser gefolgt von 200 ml gesättigter Natriumchloridlösung gewaschen, über Natriumsulfat getrocknet, filtriert und eingeengt. Es wurden 78,4 g Rohprodukt erhalten, das überwiegend aus (8-Benzyl-1,4-dioxa-spiro[4.5]dec-8-yl)-dimethyl-amin bestand und ohne zusätzliche Aufreinigung weiter umgesetzt wurde.

200 ml Salzsäure (32 m%) wurden mit 120 ml Wasser verdünnt, in diese ca. sechsmolare Salzsäure 78,4 g rohes (8-Benzyl-1,4-dioxa-spiro[4.5]dec-8-yl)-dimethyl-amin gegeben und 24 Stunden gerührt. Das Reaktionsgemisch wurde dreimal mit je 100 ml Diethylether gewaschen, unter Eisbadkühlung mit 100 ml Natronlauge (32 m%) alkalisch gestellt (pH > 10) und dreimal mit je 100 ml Dichlormethan extrahiert. Die Extrakte wurden vereinigt, über Natriumsulfat getrocknet, filtriert und eingeengt. Es wurden 50,4 g 4-Benzyl-4-dimethylamino-cyclohexanon erhalten.

40,0 g 4-Benzyl-4-dimethylamino-cyclohexanon wurden in 250 ml Isopropanol suspendiert, bei Eisbadkühlung 19,9 g Natriumboranat zugegeben und unter Erwärmung auf RT über Nacht gerührt. Es wurden 65 ml Phospharpuffer (pH 7, Merck-Darmstadt) zugetropft und der Ansatz eingeengt. Der Rückstand wurde mit Wasser und Dichlormethan aufgenommen und mit Kaliumhydroxid alkalisch gestellt. Die Phasen wurden getrennt und dreimal mit Dichlormethan extrahiert. Die vereinigten organischen Phasen wurden über Natriumsulfat getrocknet, filtriert und eingeengt. Es wurden 37,7 g 4-Benzyl-4-dimethylaminocyclohexanol erhalten.

2,65 g 4-Benzyl-4-dimethytarninocyclohexanol wurden in 20 ml Dimethylformanid gelöst, 1,40 g Kalium tert.-butylat zugegeben und 45 Minuten gerührt, bevor 2,0 g 3-Fluorbenzylchlorid innerhalb 30 Minuten zugetropft wurden. Nach Rühren über Nacht wurde der Ansatz auf 25 ml Eiswasser gegeben und wiederholt mit Ethylacetat extrahiert. Die vereinigten Extarkte wurden mit gesättigter Natriumchloridlösung gewaschen, über Natriumsulfat getrocknet, filtriert und eingeengt. Das erhaltene Rohprodukt (3,69 g) wurde mit Hexan/Ethylacetat (V:V = 1:1) an Kieselgel getrennt. Es wurden 1,96 g [1-Benzyl-4-(3-fluor-benzyloxy)-cyclohexyl]dimethylamin erhalten, die, wie für Beispiel 1 beschrieben, mit Chlortrimethylsllan und Wasser in 2-Butanon in 1,13 g des korrespondierenden Hydrochlorids überführt wurden (weißer

Feststoff).

Beispiel 6: [1-Benzyl-4-(2-fluorbenzyloxy)cyclohexyl]dimethylamin

2,00 g 4-Benzyl-4-dimethylaminocyclohexanol wurden in 20 ml Dimethylformanid gelöst, 1,06 g Kalium tert.-butylat zugegeben und 45 Minuten gerührt, bevor 1,36 g 3-Fluorbenzylchlorid innerhalb 30 Minuten zugetropft wurden. Nach Rühren über Nacht wurde der Ansatz auf 25 ml Eiswasser gegeben und wiederholt mit Ethylacetat extrahiert. Die vereinigten Extarkte wurden mit gesättigter Natriumchloridlösung gewaschen, über Natriumsulfat getrocknet, filtriert und eingeengt. Das erhaltene Rohprodukt (2,98 g) wurde mit Hexan/Ethylacetat (V:V = 1:1) an Kieselgel getrennt. Es wurden 913 mg [1-Benzyl-4-(2-fluorbenzyloxy)cydohexyl]dimethylamin erhalten, die, wie für Beispiel 1 beschrieben, mit Chlortrimethylsilan und Wasser in 2-Butanon in 620 mg des korrespondierenden Hydrochlorids überführt wurden (weißer Feststoff).

Beispiel 7: [1-Benzyl-4-(4-fluor-benzyloxy)-cyclohexyl]dimethylamin

2,00 g 4-Benzyl-4-dimethylaminocyclohexanol wurden in 20 ml Dimethylformanid gelöst, 1,06 g Kalium tert.-butylat zugegeben und 45 Minuten gerührt, bevor 1,36 g 4-Fluorbenzylchlorid innerhalb 30 Minuten zugetropft wurden. Nach Rühren über Nacht wurde der Ansatz auf 25 ml Eiswasser gegeben und wiederholt mit Ethylacetat extrahiert. Die vereinigten Extarkte wurden mit gesättigter Natriumchloridlösung gewaschen, über Natriumsulfat getrocknet, filtriert und eingeengt. Das erhaltene Rohprodukt (2,78 g) wurde mit Hexan/Ethylacetat (V:V = 1:1) an Kieselgel getrennt. Es wurden 1,58 g [1-Benzyl-4-(4-fluorbenzyloxy)cyclohexyl]dimethylamin erhalten, die, wie für Beispiel 1 beschrieben, mit Chlortrimethylsilan und Wasser in 2-Butanon in 890 mg des korrespondierenden Hydrochlorids überführt wurden (weißer Feststoff).

Beispiel 8: [4-(2-Fluorbenzyloxy)-1-phenylcyclohexyl)dimethylamin

2.00 g 4-Dimethylamino-4-phenylcyclohexanol wurden in 20 ml Dimethylformanid gelöst, 1,13 g Kalium tert.-butylatzugegeben und 45 Minuten gerührt, bevor 1,45 g 2-Fluorbenzylchlorid innerhalb 15 Minuten zugetropft wurden. Nach Rühren über Nacht wurde der Ansatz auf 25 ml Eiswasser gegeben und wiederholt mit Ethylacetat extrahiert. Die vereinigten Extarkte wurden mit gesättigter Natriumchloridlösung gewaschen, über Natriumsulfat getrocknet, filtriert und eingeengt. Das erhaltene Rohprodukt (2,61 g) wurde mit Hexan/Diethylether (V:V =1:1) an Kieselgel getrennt. Es wurden 1,08 g [4-(2-Fluorbenzyloxy)-1-phenylcyclohexyl]dimethylamin erhalten, die, wie für Beispiel 1 beschrieben, mit Chlortrimethylsilan und Wasser in 2-Butanon in 970 mg des korrespondierenden Hydrochlorids überführt wurden.

Beispiel 9: [4-(3-Fluorbenzyloxy)-1-phenylcyclohexyl]dimethylamin

2,00 g 4-Dimethylamino-4-phenylcyclohexanol wurden in 20 ml Dimethylformanid gelöst, 1,13 g Kalium tert.-butylat zugegeben und 45 Minuten gerührt, bevor 1,45 g 3-Fluorbenzylchlorid innerhalb 15 Minuten zugetropft wurden. Nach Rühren über Nacht wurde der Ansatz auf 25 ml Eiswasser gegeben und wiederholt mit Ethylacetat extrahiert. Die vereinigten Extarkte wurden mit gesättigter Natriumchloridlösung gewaschen, über Natriumsulfat getrocknet, filtriert und eingeengt. Das erhaltene Rohprodukt (2,71 g) wurde mit Hexan/Diethylether (V:V = 1:1) an Kieselgel getrennt. Es wurden 996 mg [4-(3-Fluorbenzyloxy)-1-phenylcyclohexyl]dimethylamin erhalten, die, wie für Beispiel 1 beschrieben, mit Chlortrimethylsilan und Wasser in 2-Butanon in 720 mg des korrespondierenden Hydrochlorids überführt wurden.

Beispiel 10: [4-(4-Fluorbenzyloxy)-1-phenylcyclohexyl]dimethylamin

2,00 g 4-Dimethylamino-4-phenylcyclohexanol wurden in 20 ml Dimethylformanid gelöst, 1,13 g Kalium tert.-butylat zugegeben und 45 Minuten gerührt, bevor 1,45 g 3-Fluorbenzylchlorid innerhalb 15 Minuten zugetropft wurden. Nach Rühren über Nacht wurde der Ansatz auf 25 ml Eiswasser gegeben und wiederholt mit Ethylacetat extrahiert. Die vereinigten Extarkte wurden mit gesättigter Natriumchloridlösung gewaschen, über Natriumsulfat getrocknet, filtriert und eingeengt. Das erhaltene Rohprodukt (2,66 g) wurde mit Hexan/Diethylether (V:V =1:1) an Kieselgel getrennt. Es wurden 1,02 g [4-(4-Fluorbenzyloxy)-1-phenylcyclohexyl]dimethylamin erhalten, die, wie für Beispiel 1 beschrieben, mit Chlortrimethylsilan und Wasser in 2-Butanon in 1,05 g des korrespondierenden Hydrochlorids überführt wurden.

Beispiel 11: (4-Benzyloxy-1-thiophen-2-yl-cyclohexyl)dimethylamin, unpolareres und polareres Diastereomer

4,67 g 2-Iodthiophen wurden in 20 ml Tetrahydrofuran gelöst und bei Eisbadkühlung 7,26 ml zweimolarer Isopropylmagnesiumchloridlösung in Tetrahydrofuran unter Stickstoffatmosphäre zugetropft Nach einer Stunde wurden

2,50 g 4-Benzyloxy-1-dimethylaminocyclohexancarbonitril zugetropft, gelöst in 10 ml Tetrahydrofuran. Nach Rühren über Nacht unter Erwärmung auf RT wurde erneut im Eisbad gekühlt und 25 ml kalter Ammoniumchloridlösung (20 m%) zugegeben. Die Phasen wurden getrennt, dreimal mit je 40 ml Diethylether extrahiert, die vereinigten organischen Phasen über Natriumsulfat getrocknet, filtriert und eingeengt. Das erhaltene Rohprodukt (3,82 g) wurde mit Diethylether an Kieselgel getrennt. Es wurden 1,59 g des unpolareren und 260 mg des polareren Diastereomers von (4-Benzyloxy-1-thiophen-2-yl-cyclohexyl)dimethylamin erhalten, die, wie für Beispiel 1 beschrieben, mit Chlortrimethylsilan und Wasser in 2-Butanon in 1, 11 g (Beispiel 11) bzw. 210 mg (Beispiel 12) der korrespondierenden Hydrochloride überführt wurden.

Referenzbeispiele 13 & 14: (1 H-Indol-3-yl)-essigsäure 4-dimethylamino-4-phenyl-cyclohexyl ester Hydrochlorid, unpolareres und polareres Diastereomer

175 mg (1H-Indol-3-yl)-essigsäure und 219 mg 4-Dimethylamino4-phenylcyclohexanol (Diastereomerengemisch analog Beispiel 4) wurden unter Argon und Eiskühlung In einem Gemisch aus 5 ml trockenem Dichlormethan und 5 ml trockenem THF mit 206 mg Dicyclohexylcarbodiimid und 12 mg 4-Dimethylaminopyridin versetzt und über Nacht gerührt. Der entstandene Feststoff wurde abfiltriert und mit wenig Diethylether gewaschen. Das erhaltene Filtrat wurde eingeengt und der Rückstand mit einem Gemisch aus je 10 ml zweimolarer Natriumhydrogencarbonatlösung und Ethylacetat fünf Minuten lang gerührt. Die Phasen wurden getrennt, die wäßrige Phase dreimal mit je 10 ml Ethylacetat extrahiert, die vereinigten organischen Phasen mit 10 ml Wasser gewaschen, über Natriumsulfat getrocknet und eingeengt. Als Rückstand wurde ein Gemisch der beiden möglichen diastereoIsomeren Ester erhalten, die mit Methanol an Kieselgel chromatographisch getrennt wurden. Es wurden 60 mg des unpolareren und 108 mg des polareren Diastereomers als farblose Öle erhalten, die, wie für Beispiel 1 beschrieben, mit Chlortrimethylsilan und Wasser in 2-Butanon in 65 mg (Beispiel 13) bzw. 118 mg (Beispiel 14) der korrespondierenden Hydrochloride überführt wurden.

## Messung der ORL1-Bindung

Die 4-Aminocyclohexanolderivate der allgemeinen Formel I wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschliessende Messung im Szintillationscounter Trilux (Wallac, Finnland), bestimmt. Die Affinität wird als $K_i$-Wert angegeben.

Von jeder dieser Verbindungen der Beispiele 1 bis 5 wurde gemäß den angegebenen molekularpharmakologischen Untersuchungen die Affinität zum ORL1-Rezeptor bestimmt. Die entsprechenden $K_i$-Werte sind in Tabelle 1 angegeben.

## Analaesieprüfung im Tail-Flick-Test an der Maus

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

Tabelle 1:

| Beispiel | $K_i$ (µM) ORL1-Bindungsassay | Antinociceptive Wirkung im Tail-Flick prozentual zur Kontrollgruppe* | |
|---|---|---|---|
| 1 | 0,09 | 74 | (1) |
| 2 | 0,40 | | |
| 3 | 0,10 | 96 | (1) |
| 4 | 0,20 | 53 | (1) |
| 5 | 0,10 | 92 | (1) |
| 6 | 0,40 | 68 | (1) |
| 7 | 0,20 | 79 | (1) |
| 8 | 0.10 | 46 | (1) |
| 9 | 0,03 | 98 | (1) |
| 10 | 0.04 | 96 | (1) |
| 11 | 0,009 | 100 | (10) |
| 12 | 0,25 | | |
| 13 ‡ | 0,003 | | |
| 14 ‡ | 0,09 | | |

‡: Referenzbeispiele
*: In Klammern ist jeweils die Dosierung in mg/kg bei intravenöser Applikation angegeben.

**Parenterale Lösung eines erfindungsgemäßen substituierten 4-Aminocyclohexanolderivats**

38 g eines der erfindungsgemäßen substituierten 4-Aminocyclohexanolderivate, hier gemäß Beispiel 11, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

**1.** Substituierte 4-Aminocyclohexanole der allgemeinen Formel I,

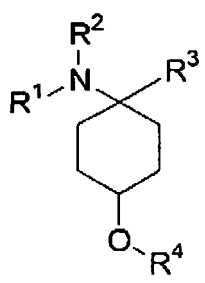

I

, worin

$R^1$ ausgewählt ist aus H; $C_{1-4}$-Alkyl, gesättigt oder ungesättigt, verzweigt oder unverzweigt, einfach oder mehrfach substituiert oder unsubstituiert; wobei $R^1$ und $R^2$ nicht beide H sein dürfen,
$R^2$ ausgewählt ist aus H; $C_{1-4}$-Alkyl, gesättigt oder ungesättigt, verzweigt oder unverzweigt, einfach oder mehrfach substituiert oder unsubstituiert
$R^3$ ausgewählt ist aus $C_{1-8}$-Alkyl, gesättigt oder ungesättigt, verzweigt oder unverzweigt, einfach oder mehrfach substituiert oder unsubstituiert; $C_{3-8}$-Cycloalkyl, einfach oder mehrfach substituiert oder unsubstituiert: Aryl,

einfach oder mehrfach substituiert oder unsubstituiert; Heterocyclyl, gesättigt oder ungesättigt, einfach oder mehrfach substituiert oder unsubstituiert; über eine $C_{1-3}$-Alkyl Brücke gebundenem Aryl oder $C_{3-8}$-Cycloalkyl, jeweils einfach oder mehrfach substituiert oder unsubstituiert oder über eine $C_{1-3}$-Alkyl Brücke gebundenem Heterocyclyl, gesättigt oder ungesättigt, einfach oder mehrfach substituiert oder unsubstituiert;
$R^4$ ausgewählt ist aus $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert; $-CHR^6R^7$, $-CHR^6-CH_2R^7$, $-CHR^6-CH_2-CH_2R^7$, $-CHR^6-CH_2-CH_2-CH_2R^7$; oder $-R^8-L-R^9$
mit $R^6$ ausgewählt aus

H; $C_{1-8}$-Alkyl, gesättigt oder ungesättigt, verzweigt oder unverzweigt, einfach oder mehrfach substituiert oder unsubstituiert; $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert; oder $C(O)O-R^{10}$-,
mit $R^{10}$ ausgewählt aus

$C_{1-7}$-Alkyl, gesättigt oder ungesättigt, verzweigt oder unverzweigt, einfach oder mehrfach substituiert oder unsubstituiert; $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert;

mit $R^7$ ausgewählt aus

H; $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert,

mit $R^8$ ausgewählt aus

$C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert,

mit L ausgewählt aus

-C(O)-NH-, -NH-C(O)-, -C(O)-O-, -O-C(O), -O-, -S- oder $-S(O)_2$-

mit $R^9$ ausgewählt aus

H; $C_{1-8}$-Alkyl, gesättigt oder ungesättigt, verzweigt oder unverzweigt, einfach oder mehrfach substituiert oder unsubstituiert; $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert;

wobei der Begriff "substituiert" im Zusammenhang mit Alkyl und Cycloalkyl die Substitution eines Wasserstoffrestes durch F, Cl, Br, I, $NH_2$, SH oder OH bedeutet, wobei unter "mehrfach substituiert" Resten zu verstehen ist, daß die Substitution sowohl an verschiedenen als auch an gleichen Atomen mehrfach mit den gleichen oder verschiedenen Substituenten erfolgt;
wobei "substituiert" im Zusammenhang mit Aryl und Heteroaryl die Substitution des Aryls oder Heteroaryls mit $R^{23}$, $OR^{23}$, einem Halogen, einem $CF_3$, einem CN, einem $NO_2$, einem $NR^{24}R^{25}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen bedeutet;

wobei
der Rest $R^{23}$ für H, einen $C_{1-10}$-Alkyl-, einen Aryl- oder Heteroaryl-oder für einen über eine $C_{1-3}$-Alkylen-Gruppe gebundenen Aryl-oder Heteroaryl-Rest steht, wobei diese Aryl und Heteroarylreste nicht selbst mit Aryl- oder Heteroaryl-Resten substituiert sein dürfen,
die Reste $R^{24}$ und $R^{25}$, gleich oder verschieden, für H, einen $C_{1-10}$-Alkyl-, einen Aryl-, einen Heteroaryl- oder einen über eine $C_{1-3}$-Alkylen-Gruppe gebundenen Aryl- oder Heteroaryl-Rest stehen, wobei diese

Aryl und Heteroarylreste nicht selbst mit Aryl- oder Heteroaryl-Resten substituiert sein dürfen, oder die Reste $R^{24}$ und $R^{25}$ zusammen $CH_2CH_2OCH_2CH_2$, $CH_2CH_2NR^{26}CH_2CH_2$ oder $(CH_2)_{3-6}$ bedeuten, und

der Rest $R^{26}$ für H, einen $C_{1-10}$-Alkyl-, einen Aryl-, oder Heteroaryl-Rest oder für einen über eine $C_{1-3}$-Alkylen-Gruppe gebundenen Aryl- oder Heteroaryl-Rest steht, wobei diese Aryl und Heteroarylreste nicht selbst mit Aryl- oder Heteroaryl-Resten substituiert sein dürfen;

in Form ihrer Racemate, ihrer reinen Enantiomere oder Diastereomere, Mischungen der Enantiomere oder Diastereomere in einem beliebigen Mischungsverhältnis; in dargestellter Form oder in Form ihrer physiologisch verträglichen Salze, oder in Form ihrer Solvate.

**2.** Substituierte 4-Aminocyclohexanole gemäß Anspruch 1, **dadurch gekennzeichnet, daß**

$R^1$ und $R^2$ Methyl bedeuten.

**3.** Substituierte 4-Aminocyclohexanole gemäß Anspruch 1, **dadurch gekennzeichnet, daß**

$R^3$ ausgewählt ist aus $C_{3-8}$-Cycloalkyl, einfach oder mehrfach substituiert oder unsubstituiert; Aryl, einfach oder mehrfach substituiert oder unsubstituiert; Heterocyclyl, gesättigt oder ungesättigt, einfach oder mehrfach substituiert oder unsubstituiert; über eine $C_{1-3}$-Alkyl Brücke gebundenem Aryl oder $C_{3-8}$-Cycloalkyl, jeweils einfach oder mehrfach substituiert oder unsubstituiert oder über eine $C_{1-3}$-Alkyl Brücke gebundenem Heterocyclyl, gesättigt oder ungesättigt, jeweils einfach oder mehrfach substituiert oder unsubstituiert.

**4.** Substituierte 4-Aminocyclohexanole gemäß Anspruch 1, **dadurch gekennzeichnet, daß**

$R^4$ ausgewählt ist aus $C_{3-8}$-Cycloalkyl, Aryl oder Heteroaryl, jeweils unsubstituiert oder einfach oder mehrfach substituiert; oder $-R^8-L-R^9$.

**5.** Substituierte 4-Aminocyclohexanole gemäß Anspruch 4, **dadurch gekennzeichnet, daß**

$R^8$ ausgewählt ist aus

Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,

L ausgewählt aus

-C(O)-NH-, -NH-C(O)-, -C(O)-O-, -O-C(O)-, -O-, -S- oder -S(Oh-,

und/oder $R^9$ ausgewählt ist aus

Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Pyrimidinyl, Chinolinyl, Isochinolinyl, Phthalazinyl oder Chinazolinyl, jeweils unsubstituiert oder einfach oder mehrfach substituiert,

**6.** Substituierte 4-Aminocyclohexanole gemäß Anspruch 1, **dadurch gekennzeichnet, daß**

$R^4$ ausgewählt ist aus $-CHR^6R^7$, $-CHR^6-CH_2R^7$, $-CHR^6-CH_2-CH_2R^7$, $-CHR^6-CH_2-CH_2-CH_2R^7$.

**7.** Substituierte 4-Aminocyclohexanole gemäß Anspruch 6, **dadurch gekennzeichnet, daß**

$R^6$ ausgewählt ist aus

H, $C_{1-4}$-Alkyl, gesättigt oder ungesättigt, verzweigt oder unverzweigt, einfach oder mehrfach substituiert oder unsubstituiert; oder C(O)O $R^{10}$

mit $R^{10}$ ausgewählt aus $C_{1-4}$-Alkyl, gesättigt oder ungesättigt, verzweigt oder unverzweigt, einfach oder mehrfach substituiert oder unsubstituiert.

**8.** Substituierte 4-Aminocyclohexanole gemäß einem der Ansprüche 6 bis 7, **dadurch gekennzeichnet, daß**

$R^7$ ausgewählt ist aus $C_{3-8}$-Cycloalkyl, Aryl oder Heteroaryl, jeweils unsubstituiert oder einfach oder mehrfach substituiert.

**9.** Substituierte 4-Aminocyclohexanole gemäß einem der Ansprüche 1 bis 8, **dadurch gekennzeichnet, daß** sie ausgewählt sind aus der folgenden Gruppe:

- trans-(4-Benzyloxy-1-phenylcyclohexyl)dimethylamin
- cis-{4-Benzyloxy-1-pheny(cyclohexyl)dimethyfamin
- trans-(1-Benzyl-4-benzyloxycyclohexyl)dimethylamin
- cis-(1-Benzyl-4-benzyloxycyclohexyl)dimethylamin
- trans-[4-Benzyloxy-1-(2-methylbenzyl)cyclohexyl]dimethylamin
- cis-[4-Benzyloxy-1-(2-methylbenzyl)cydohexyl]dimethylamin
- cis-[4-(2-Fluorbenzyloxy)-1-phenylcyclohexyl]dimethylamin
- cis-[1-Benzyl-4-(3-fluorbenzyloxy)cyclohexyl]dimethylamin
- cis-[1-Benzyl-4-(2-fluorbenzyloxy)cyclohexyl]dimethylamin
- cis-[1-Benzyl-4-(4-fluorbenzyloxy)cyclohexyl]dimethylamin
- trans-[4-(2-Fluorbenzyloxy)-1-phenylcyclohexyl]dimethylamin
- trans-[4-(3-Fluorbenzyloxy)-1-phenylcyclohexyl]dimethylamin
- trans-[4-(4-Fluorbenzyloxy)-1-phenylcyclohexyl]dimethylamin
- trans-[4-(4-Fluorbenzyloxy)-1-phenethylcyclohexyl]dimethylamin
- trans-[4-(3-Fluor-benzyloxy)-1-phenethylcydohexyl]dimethylamin,

in Form ihrer Racemate, ihrer reinen Enantiomere oder Diastereomere, Mischungen der Enantiomere oder Diastereomere in einem beliebigen Mischungsverhältnis; in dargestellter Form oder in Form ihrer physiologisch verträglichen Salze, oder in Form ihrer Solvate.

**10.** Arzneimittel enthaltend wenigstens ein substituiertes 4-Aminocyclohexanol gemäß einem der Ansprüche 1 bis 9 in Form ihrer Racemate, ihrer reinen Enantiomere oder Diastereomere, Mischungen der Enantiomere oder Diastereomere in einem beliebigen Mischungsverhältnis; in dargestellter Form oder in Form ihrer physiologisch verträglichen Salze, oder in Form ihrer Solvate.

**11.** Arzneimitteln gemäß Anspruch 10, **dadurch gekennzeichnet, daß** das Arzneimittel neben wenigstens einem substituierten 4-Aminocyclohexanol noch ein Opioid enthält.

**12.** Verwendung eines substituierten 4-Aminocyclohexanols gemäß einem der Ansprüche 1 bis 9 in Form ihrer Racemate, ihrer reinen Enantiomere oder Diastereomere, Mischungen der Enantiomere oder Diastereomere in einem beliebigen Mischungsverhältnis; in dargestellter Form oder in Form ihrer physiologisch verträglichen Salze, oder in Form ihrer Solvate zur Herstellung eines Arzneimittels zur Behandlung von Schmerz.

**13.** Verwendung eines substituierten 4-Aminocyclohexanols gemäß einem der Ansprüche 1 bis 9 in Form ihrer Racemate, ihrer reinen Enantiomere oder Diastereomere, Mischungen der Enantiomere oder Diastereomere in einem beliebigen Mischungsverhältnis; in dargestellter Form oder in Form ihrer physiologisch verträglichen Salze, oder in Form ihrer Solvate zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese und/oder

Anxiolyse.

**14.** Verfahren zur Herstellung eines substituierten 4-Aminocyclohexanols gemäß Anspruch 1 mit folgenden Schritten:

a. ein mit den Gruppen $S^1$ und $S^2$ geschütztes Cyclohexan-1,4-dion gemäß Formel IV wird in Gegenwart einer Verbindung der Formel $HNR^{01}R^{02}$ mit einem Cyanid zu einem geschützten N-substituierten 1-Amino-4-oxo-cyclo-hexancarbonitrilderivat gemäß Formel V umgesetzt;

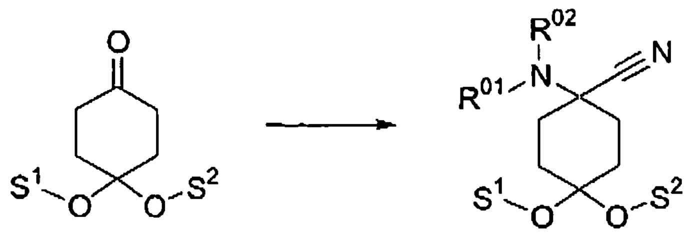

IV V

b. das Aminonitril gemäß Formel V wird mit metallorganischen Reagenzien, bevorzugt Grignard- oder Organolithiumreagenzien, der Formel Metall-$R^3$ umgesetzt, so daß eine Verbindung gemäß Formel VI entsteht;

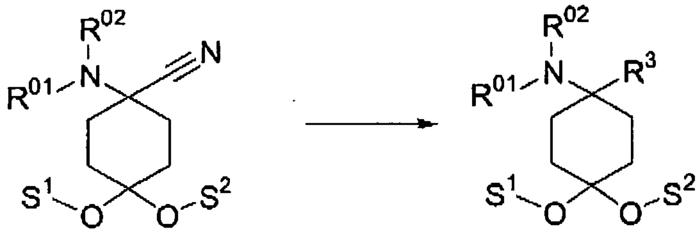

V VI

c. an der Verbindung gemäß Formel VI werden die Schutzgruppen $S^1$ und $S^2$ abgespalten, so daß ein 4-substituiertes 4-Aminocyclohexanonderivat gemäß Formel VII entsteht;

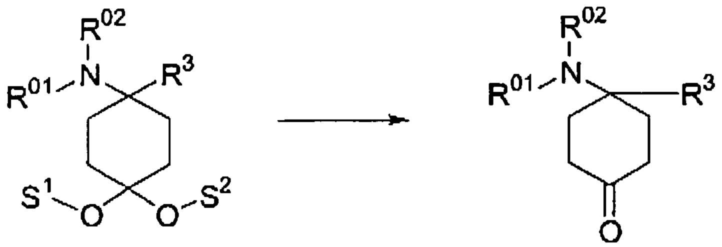

VI VII

d. das 4-substituierte 4-Aminocyclohexanonderivat gemäß Formel VII wird mit einem Reduktionsmittel wie beispielsweise Natriumborhydrid, Natriumcyanoborhydrid, Natriumtriacetoxyborhydrid, Lithiumaluminiumhydrid, Diisobutylaluminiumhydrid, einem komplexen Analogon dieser Verbindungen bei Temperaturen zwischen -70 °C und +110 °C, oder edelmetallkatalysiert mit Wasserstoff zu einem 4-Aminocyclohexanolderivat gemäß Formel VIII umgesetzt;

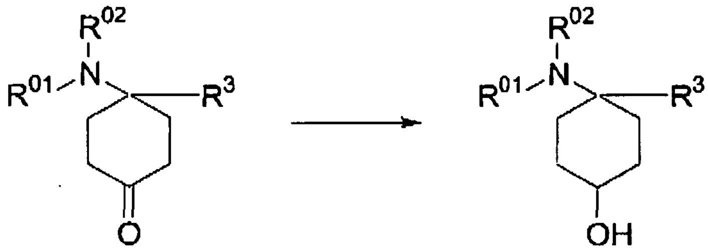

VII VIII

e. das 4-substituierte 4-Aminocyclohexanolderivat gemäß Formel VIII wird anschließend in Gegenwart einer anorganischen, metallorganischen oder organischen Base mit einem Alkyl-, Acyl- oder Arylbromid, -chlorid, -iodid, - triflat oder mit einem mit einer anderen Abgangsgruppe X versehenen Alkan, Alkylsäure oder Aromaten $R^4X$ zu einer Verbindung gemäß Formel I umgesetzt,

wobei $R^1$, $R^2$, $R^3$ und $R^4$ die in Anspruch 1 angegebene Bedeutung haben und $R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert.

**15.** Alternatives Verfahren zur Herstellung eines substituierten 4-Aminocyclohexanols gemäß Anspruch 1 mit folgenden Schritten:

a. ein mit den Gruppen $S^1$ und $S^2$ geschütztes Cyclohexan-1,4-dion gemäß Formel IV mit einem Reduktionsmittel wie beispielsweise Natriumborhydrid, Natriumcyanoborhydrid, Natriumtriacetoxyborhydrid, Lithiumaluminiumhydrid, Diisobutylaluminiumhydrid, einem komplexen Analogon dieser Verbindungen bei Temperaturen zwischen -70 °C und +110 °C, oder edelmetallkatalysiert mit Wasserstoff zu einem geschützten 4-Hydroxycyclohexanonderivat gemäß Formel XIII umgesetzt;

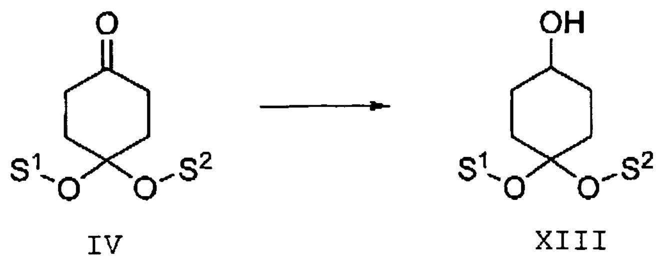

IV XIII

b. das anschließend in Gegenwart einer anorganischen, metallorganischen oder organischen Base mit einem Alkyl- oder Arylbromid, -chlorid, -iodid, -triflat oder mit einem mit einer anderen Abgangsgruppe X versehenen Alkan oder Aromaten $R^4X$ zu einer Verbindung gemäß Forme XIV umgesetzt wird;

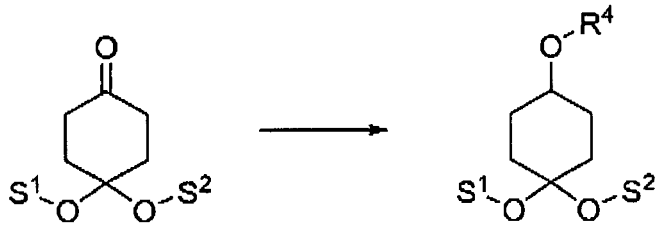

XIII XIV

c. an der Verbindung gemäß Formel XIV werden die Schutzgruppen $S^1$ und $S^2$ abgespalten, so daß eine Verbindung gemäß Formel XV entsteht;

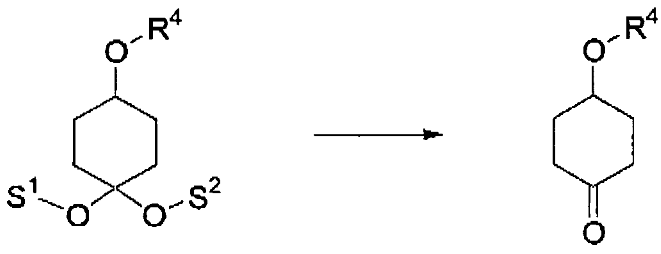

XIV XV

d. die Verbindung Formel XV wird in Gegenwart einer Verbindung der Formel $HNR^{01}R^{02}$ mit Cyanid zu einem α-Aminonitrilderivat der Formel XVI umgesetzt,

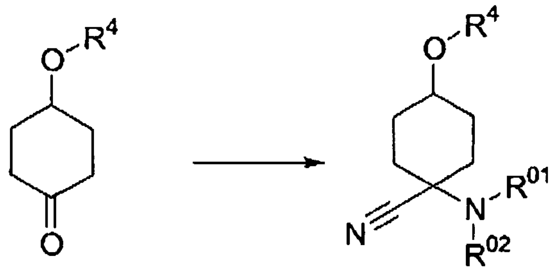

XV XVI

e. das α-Aminonitrilderivat der Formel XVI wird mit metallorganischen Reagenzien der Formel Metall-$R^3$ zu einer Verbindung gemäß Formel I umgesetzt,

wobei $R^1$, $R^2$, $R^3$ und $R^4$ die in Anspruch 1 angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert.

## Claims

**1.** Substituted 4-aminocyclohexanols of the general formula I

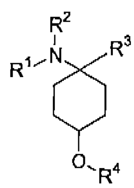

I

wherein

$R^1$ is selected from H; $C_{1-4}$-alkyl, saturated or unsaturated, branched or unbranched, mono- or poly-substituted

or unsubstituted; wherein $R^1$ and $R^2$ may not both be H;
$R^2$ is selected from H; $C_{1-4}$-alkyl, saturated or unsaturated, branched or unbranched, mono- or poly-substituted or unsubstituted;
$R^3$ is selected from $C_{1-8}$-alkyl, saturated or unsaturated, branched or unbranched, mono- or poly-substituted or unsubstituted; $C_{3-5}$-cycloalkyl, mono- or poly-substituted or unsubstituted; aryl, mono- or poly-substituted or unsubstituted; heterocyclyl, saturated or unsaturated, mono- or poly-substituted or unsubstituted; aryl or $C_{3-8}$-cycloalkyl bonded *via* a $C_{1-3}$-alkyl bridge, in each case mono- or poly-substituted or unsubstituted; or heterocyclyl bonded *via* a $C_{1-3}$-alkyl bridge, saturated or unsaturated, mono- or poly-substituted or unsubstituted.
$R^4$ is selected from $C_{3-8}$-cycloalkyl, mono- or poly-substituted or unsubstituted; aryl, mono- or poly-substituted or unsubstituted; heterocyclyl, saturated or unsaturated, mono- or poly-substituted or unsubstituted; $-CHR^6R^7$, $-CHR^6-CH_2R^7$, $-CHR^6-CH_2-CH_2R^7$, $-CHR^6-CH_2-CH_2-CH_2R^7$; or $-R^8-L-R^9$
where $R^6$ is selected from

H; $C_{1-8}$-alkyl, saturated or unsaturated, branched or unbranched, mono- or poly-substituted or unsubstituted; $C_{3-8}$-cycloalkyl, mono- or poly-substituted or unsubstituted; aryl, mono- or poly-substituted or unsubstituted; heterocyclyl, saturated or unsaturated, mono- or poly-substituted or unsubstituted; or $C(O)O-R^{10}$;
where $R^{10}$ is selected from

$C_{1-7}$-alkyl, saturated or unsaturated, branched or unbranched, mono-or poly-substituted or unsubstituted; $C_{3-8}$-cycloalkyl, mono- or poly-substituted or unsubstituted; aryl, mono- or poly-substituted or unsubstituted; heterocyclyl, saturated or unsaturated, mono- or poly-substituted or unsubstituted;

where $R^7$ is selected from

H; $C_{3-6}$-cycloalkyl, mono- or poly-substituted or unsubstituted; aryl, mono- or poly-substituted or unsubstituted; heterocyclyl, saturated or unsaturated, mono- or poly-substituted or unsubstituted,

where $R^8$ is selected from

$C_{3-8}$-cycloalkyl, mono- or poly-substituted or unsubstituted; aryl, mono- or poly-substituted or unsubstituted; heterocyclyl, saturated or unsaturated, mono- or poly-substituted or unsubstituted,

where L is selected from

-C(O)-NH-, -NH-C(O)-, -C(O)-O-, -O-C(O)-, -O-, -S- or-$S(O)_2$-,

where $R^9$ is selected from

H; $C_{1-8}$-alkyl, saturated or unsaturated, branched or unbranched, mono- or poly-substituted or unsubstituted; $C_{3-8}$-cycloalkyl, mono- or poly-substituted or unsubstituted; aryl, mono- or poly-substituted or unsubstituted; heterocyclyl, saturated or unsaturated, mono- or poly-substituted or unsubstituted;

wherein the term "substituted" in connection with alkyl and cycloalkyl denotes the substitution of a hydrogen radical by F, Cl, Br, I, $NH_2$, SH or OH, where "polysubstituted" radicals are to be understood as meaning that the substitution occurs several times with the same or different substituents both on different and on the same atoms;
wherein "substituted" in connection with aryl and heteroaryl means the substitution of the aryl or heteroaryl by $R^{23}$, $OR^{23}$, a halogen, a $CF_3$, a CN, an $NO_2$, an $NR^{24}R^{25}$, a $C_{1-6}$-alkyl (saturated), a $C_{1-6}$-alkoxy, a $C_{3-8}$-cycloalkoxy, a $C_{3-8}$-cycloalkyl or a $C_{2-6}$-alkylene;

wherein
the radical $R^{23}$ represents H, a $C_{1-10}$-alkyl radical, an aryl or heteroaryl radical or an aryl or heteroaryl radical bonded *via* a $C_{1-3}$-alkylene group, wherein these aryl and heteroaryl radicals may not themselves be substituted by aryl or heteroaryl radicals.
the radicals $R^{24}$ and $R^{25}$, which are identical or different, represent H, a $C_{1-10}$-alkyl radical, an aryl radical, a heteroaryl radical or an aryl or heteroaryl radical bonded via a $C_{1-3}$-alkylene group, wherein these aryl and heteroaryl radicals may not themselves be substituted by aryl or heteroaryl radicals,
or the radicals $R^{24}$ and $R^{25}$ together represent $CH_2CH_2OCH_2CH_2$, $CH_2CH_2NR^{26}CH_2CH_2$ or $(CH_2)_{3-6}$, and

the radical $R^{26}$ represents H, a $C_{1-10}$-alkyl radical, an aryl or heteroaryl radical or an aryl or heteroaryl radical bonded via a $C_{1-3}$-alkylene group, wherein these aryl and heteroaryl radicals may not themselves be substituted by aryl or heteroaryl radicals:

in the form of their racemates, their pure enantiomers or diastereoisomers, mixtures of the enantiomers or diastereoisomers in any mixing ratio; in the prepared form or in the form of their physiologically acceptable salts, or in the form of their solvates.

**2.** Substituted 4-aminocyclohexanols according to claim 1, **characterised in that**

$R^1$ and $R^2$ represent methyl.

**3.** Substituted 4-aminocyclohexanols according to claim 1, **characterised in that**

$R^3$ is selected from $C_{3-8}$-cycloalkyl, mono- or poly-substituted or unsubstituted; aryl, mono- or poly-substituted or unsubstituted; heterocyclyl, saturated or unsaturated, mono- or poly-substituted or unsubstituted; aryl or $C_{3-8}$-cycloalkyl bonded via a $C_{1-3}$-alkyl bridge, in each case mono- or poly-substituted or unsubstituted; or heterocyclyl bonded *via* a $C_{1-3}$-alkyl bridge, saturated or unsaturated, in each case mono- or poly-substituted or unsubstituted.

**4.** Substituted 4-aminocyclohexanols according to claim 1, **characterised in that**

$R^4$ is selected from $C_{3-8}$-cycloalkyl, aryl or heteroaryl, in each case unsubstituted or mono- or poly-substituted; or-$R^8$-L-$R^9$.

**5.** Substituted 4-aminocyclohexanols according to claim 4, **characterised in that**

$R^8$ is selected from

indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl, in each case unsubstituted or mono- or poly-substituted,

L is selected from

-C(O)-NH-, -NH-C(O)-, -C(O)-O-, -O-C(O)-, -O-, -S- or-$S(O)_2$,

and/or $R^9$ is selected from

indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, pyrimidinyl, quinolinyl, isoquinolinyl, phthalazinyl or quinazolinyl, in each case unsubstituted or mono- or poly-substituted.

**6.** Substituted 4-aminocyclohexanols according to claim 1, **characterised in that**

$R^4$ is selected from $-CHR^6R^7$, $-CHR^6-CH_2R^7$, $-CHR^6-CH_2-CH_2R^7$, $-CHR^6-CH_2-CH_2-CH_2R^7$.

**7.** Substituted 4-aminocyclohexanols according to claim 6, **characterised in that**

$R^6$ is selected from

H, $C_{1-4}$-alkyl, saturated or unsaturated, branched or unbranched, mono- or poly-substituted or unsubstituted; or $C(O)OR^{10}$

where $R^{10}$ is selected from $C_{1-4}$-alkyl, saturated or unsaturated, branched or unbranched, mono- or poly-substituted or unsubstituted.

**8.** Substituted 4-aminocyclohexanols according to any one of claims 6 to 7, **characterised in that**

$R^7$ is selected from $C_{3-8}$-cycloalkyl, aryl or heteroaryl, in each case unsubstituted or mono- or poly-substituted.

**9.** Substituted 4-aminocyclohexanols according to any one of claims 1 to 8, **characterised in that** they are selected from the following group:

- trans-(4-benzyloxy-1-phenylcyclohexyl)dimethylamine
- cis-(4-benzyloxy-1-phenylcyclohexyl)dimethylamine
- trans-(1-benzyl-4-benzyloxycyclohexyl)dimethylamine
- cis-(1-benzyl-4-benzyloxycyclohexyl)dimethylamine
- trans-[4-benzyloxy-1-(2-methylbenzyl)cyclohexyl]dimethylamine
- cis-[4-benzyloxy-1-(2-methylbenzyl)cyclohexyl]dimethylamine
- cis-[4-(2-fluorobenzyloxy)-1-phenylcyclohexyl]dimethylamine
- cis-[1-benzyl-4-(3-fluorobenzyloxy)cyclohexyl]dimethylamine
- cis-[1-benzyl-4-(2-fluorobenzyloxy)cyclohexyl]dimethylamine
- cis-[1-benzyl-4-(4-fluorobenzyloxy)cyclohexyl]dimethylamine
- trans-[A-(2-fluorobenzyloxy)-1-phenylcyclohexyl]dimethylamine
- trans-[4-(3-fluorobenzyloxy)-1-phenylcyclohexyl]dimethylamine
- trans-[4-(4-fluorobenzyloxy)-1-phenylcyclohexyl]dimethylamine
- trans-[4-(4-fluorobenzyloxy)-1-phenethylcyclohexyl]dimethylamine
- trans-[4-(3-fluorobenzyloxy)-1-phenethylcyclohexyl]dimethylamine,

in the form of their racemates, of their pure enantiomers or diastereoisomers, mixtures of the enantiomers or diastereoisomers in any mixing ratio; in the prepared form or in the form of their physiologically acceptable salts, or in the form of their solvates.

**10.** Medicament comprising at least one substituted 4-aminocyclohexanol according to any one of claims 1 to 9 in the form of its racemate, its pure enantiomers or diastereoisomers, mixtures of the enantiomers or diastereoisomers in any mixing ratio: in the prepared form or in the form of its physiologically acceptable salts, or in the form of its solvates.

**11.** Medicament according to claim 10, **characterised in that** the medicament, in addition to comprising at least one substituted 4-aminocyclohexanol, also comprises an opioid.

**12.** Use of a substituted 4-aminocyclohexanol according to any one of claims 1 to 9 in the form of its racemates, its pure enantiomers or diastereoisomers, mixtures of the enantiomers or diastereoisomers in any mixing ratio; in the prepared form or in the form of its physiologically acceptable salts, or in the form of its solvates, in the preparation of a medicament for the treatment of pain.

**13.** Use of a substituted 4-aminocyclohexanol according to any one of claims 1 to 9 in the form of its racemates, its pure enantiomers or diastereoisomers, mixtures of the enantiomers or diastereoisomers in any mixing ratio; in the prepared form or in the form of its physiologically acceptable salts, or in the form of its solvates, in the preparation of a medicament for the treatment of anxiety, stress and syndromes associated with stress, depression, epilepsy, Alzheimer's disease, senile dementia, general cognitive dysfunctions, learning and memory difficulties (as a nootropic agent), withdrawal symptoms, alcohol and/or drug and/or medicament abuse and/or dependency, sexual dysfunctions, cardiovascular diseases, hypotension, hypertension, tinnitus, pruritus, migraine, impaired hearing, deficient intestinal motility, impaired food intake, anorexia, obesity, locomotor disorders, diarrhoea, cachexia, urinary incontinence or as a muscle relaxant, anticonvulsive or anaesthetic or for co-administration on treatment with an opioid analgesic or with an anaesthetic, for diuresis or antinatriuresis and/or anxiolysis.

**14.** Process for the preparation of a substituted 4-aminocyclohexanol according to claim 1, comprising the following steps:

a. a cyclohexane-1,4-dione, protected with groups $S^1$ and $S^2$. according to formula IV is reacted with a cyanide in the presence of a compound of the formula $HNR^{01}R^{02}$ to give a protected N-substituted 1-amino-4-oxo-cyclohexanecarbonitrile derivative according to formula V;

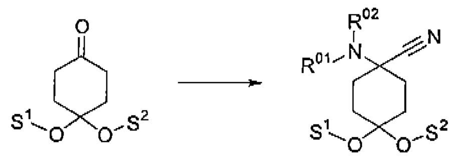

IV V

b. the aminonitrile according to formula V is reacted with organometallic reagents, preferably Grignard or organolithium reagents, of the formula metal-$R^3$, so that a compound according to formula VI is formed;

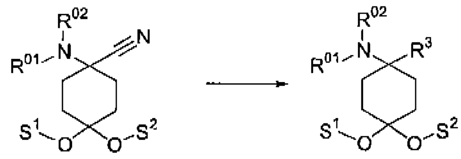

V VI

c. on the compound according to formula VI, the protecting groups $S^1$ and $S^2$ are split off, so that a 4-substituted 4-aminocyclohexanone derivative according to formula VII is formed;

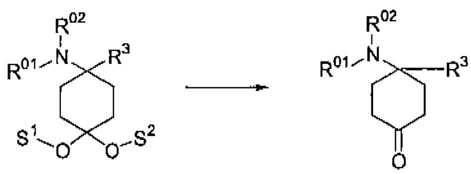

VI VII

d. the 4-substituted 4-aminocyclohexanone derivative according to formula VII is is reacted with a reducing agent such as, for example, sodium borohydride, sodium cyanoborohydride, sodium triacetoxyborohydride, lithium aluminium hydride, diisobutylaluminium hydride, a complex analogue of these compounds, at temperatures of from -70°C to +110°C, or with noble metal catalysis with hydrogen, to give a 4-aminocyclohexanol derivative according to formula VIII;

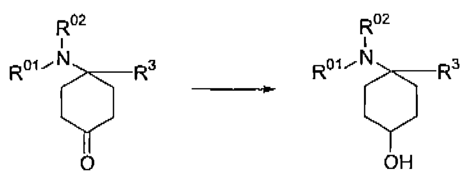

VII VIII

e. the 4-substituted 4-aminocyclohexanol derivative according to formula VIII is then reacted in the presence of an inorganic, organometallic or organic base with an alkyl, acyl or aryl bromide, chloride, iodide or triflate or with an alkane, alkyl acid or aromatic compound $R^4X$ provided with a different leaving group X to give a compound according to formula I,

wherein $R^1$, $R^2$, $R^3$ and $R^4$ are as defined in claim 1
and
$R^{01}$ and $R^{02}$ are selected independently of one another from H; H provided with a protecting group; $C_n$-alkyl or $C_{3-6}$-cycloalkyl, in each case saturated or unsaturated, branched or unbranched, mono- or poly-substituted or unsubstituted; aryl or heteroaryl, in each case mono- or poly-substituted or unsubstituted; or aryl, $C_{3-8}$-cycloalkyl or heteroaryl bonded via $C_{1-3}$-alkylene, in each case mono- or poly-substituted or unsubstituted.

**15.** Alternative process for the preparation of a substituted 4-aminocyclohexanol according to claim 1, comprising the

following steps:

a. a cyclohexane-1,4-dione, protected with groups $S^1$ and $S^2$, according to formula IV is reacted with a reducing agent such as, for example, sodium borohydride, sodium cyanoborohydride, sodium triacetoxyborohydride, lithium aluminium hydride, diisobutylaluminium hydride, a complex analogue of these compounds, at temperatures of from -70°C to +110°C, or with noble metal catalysis with hydrogen, to give a protected 4-hydroxycyclohexanone derivative according to formula XIII;

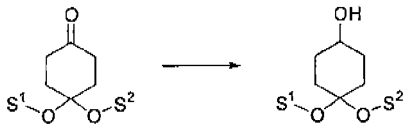

IV XIII

b. which is then reacted in the presence of an inorganic, organometallic or organic base with an alkyl or aryl bromide, chloride, iodide or triflate or with an alkane or aromatic compound $R^4X$ provided with a different leaving group X, to give a compound according to formula XIV;

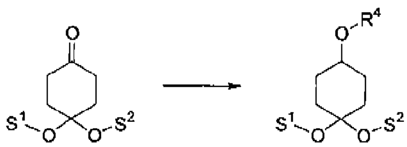

XIII XIV

c. on the compound according to formula XIV, the protecting groups $S^1$ and $S^2$ are split off, so that a compound according to formula XV is formed:

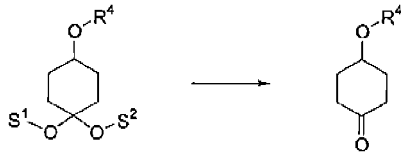

XIV XV

d. the compound of formula XV is reacted with cyanide in the presence of a compound of the formula $HNR^{01}R^{02}$ to give an α-aminonitrile derivative of formula XVI;

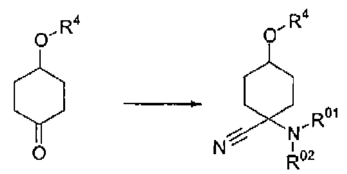

XV XVI

e. the α-aminonitrile derivative of formula XVI is reacted with organometallic reagents of the formula metal-$R^3$ to give a compound according to formula I,

wherein $R^1$, $R^2$, $R^3$ and $R^4$ are as defined in claim 1
and
$R^{01}$ and $R^{02}$ are selected independently of one another from H; H provided with a protecting group; $C_{1-8}$-alkyl or $C_{3-8}$-cycloalkyl, in each case saturated or unsaturated, branched or unbranched, mono- or poly-substituted or unsubstituted; aryl or heteroaryl, in each case mono- or poly-substituted or unsubstituted, or aryl, $C_{3-8}$-cycloalkyl or heteroaryl bonded via $C_{1-3}$-alkylene, in each case mono- or poly-substituted or unsubstituted.

## Revendications

**1.** 4-aminocyclohexanols substitués de formule générale I

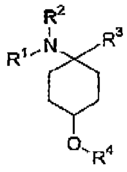

I

dans laquelle

$R^1$ est choisi parmi H ; alkyle en $C_{1-4}$, saturé ou insaturé, ramifié ou non ramifié, substitué une ou plusieurs fois ou non substitué ; $R^1$ et $R^2$ ne pouvant pas représenter tous les deux H,
$R^2$ est choisi parmi H ; alkyle en $C_{1-4}$, saturé ou insaturé, ramifié ou non ramifié, substitué une ou plusieurs fois ou non substitué ;
$R^3$ est choisi parmi alkyle en $C_{1-8}$, saturé ou insaturé, ramifié, ou non ramifié, substitué une ou plusieurs fois ou non substitué ; cycloalkyle en $C_3$-$C_8$, substitué une ou plusieurs fois ou non substitué ; aryle, substitué une ou plusieurs fois ou non substitué ; hétérocyclyle, saturé ou insaturé, substitué une ou plusieurs fois ou non substitué ; aryle ou cycloalkyle en $C_{3-8}$ relié par un pont alkyle en $C_{1-3}$, chacun substitué une ou plusieurs fois ou non substitué, ou hélérocyclyle relié par un pont allyle en $C_{1-3}$, saturé ou insaturé, substitué une ou plusieurs fois ou non substitué ;
$R^4$ est choisi parmi cycloalkyle en $C_3$-$C_8$, substitué une ou plusieurs fois ou non substitué ; aryle, substitué une ou plusieurs fois ou non substitué ; hétérocyclyle, saturé ou insaturé, substitué une ou plusieurs fois ou non substitué ; $-CHR_6R^7$, $-CHR^6-CH_2R^7$, $-CHR^6-CH_2-CH_2R^7$, $- CHR^6-CH_2-CH_2-CH_2R^7$ ou $-R^8-L-R^9$;
$R^6$ étant choisi parmi

H ; alkyle en $C_{1-8}$, saturé ou insaturé, ramifié ou non ramifié, substitué une ou plusieurs fois ou non substitué ; cycloalkyle en $C_{3-8}$, substitué une ou plusieurs fois ou non substitué ; aryle, substitué une ou plusieurs fois ou non substitué ; hétérocyclyle, saturé ou insaturé, substitué une ou plusieurs fois ou non substitué ; ou $C(O)O-R^{10}$; $R^{10}$ étant choisi parmi

alkyle en $C_{1-7}$, saturé ou insaturé, ramifié ou non ramifié, substitué une ou plusieurs fois ou non substitué ; cycloalkyle en $C_{3-8}$, substitué une ou plusieurs fois ou non substitué ; aryle, substitué une ou plusieurs fois ou non substitué ; hétérocyclyle, saturé ou insaturé, substitué une ou plusieurs fois ou non substitué ;

$R^7$ étant choisi parmi

H ; cycloalkyle en $C_{3-8}$, substitué une ou plusieurs fois ou non substitué ; aryle, substitué une ou plusieurs fois ou non substitué ; hétérocyclylc, saturé ou insaturé, substitué une ou plusieurs fois ou non substitué ;

$R^8$ étant choisi parmi

cycloalkyle en $C_{3-8}$, substitué une ou plusieurs fois ou non substitué ; aryle, substitué une ou plusieurs fois ou non substitué ; hétérocyclyle, saturé ou insaturé, substitué une ou plusieurs fois ou non substitué ;

L étant choisi parmi

-C(O)-NH-, -NH-C(O)-, -C(O)-O-, -O-C(O)-, -O-, -Sou $-S(O)_2-$ ;

$R^9$ étant choisi parmi

H ; alkyle en $C_{1-8}$, saturé ou insaturé, ramifié ou non ramifié, substitué une ou plusieurs fois ou non substitué ; cycloalkyle en $C_{3-8}$, substitué une ou plusieurs fois ou non substitué ; aryle, substitué une ou plusieurs fois ou non substitué ; hétérocyclyle, saturé ou insaturé, substitué une ou plusieurs fois ou non substitué ;

le terme « substitué » au regard d'alkyle et de cycloalkyle signifiant la substitution d'un radical hydrogène par F, Cl, Br, I, $NH_2$, SH ou OH ; des radicaux « substitués plusieurs fois » signifiant que la substitution a lieu plusieurs fois aussi bien sur des atomes différents qu'identiques, avec des substituants identiques ou différents ; « substitué » au regard d'aryle et d'hétéroaryle signifiant la substitution de l'aryle ou de l'hétéroaryle avec $R^{23}$, $OR^{23}$, un halogène, un $CF_3$, un CN, un $NO_2$, un $NR^{24}R^{25}$, un alkyle en $C_{1-6}$ (saturé), un alcoxy en $C_{1-6}$, un cycloalcoxy en $C_{3-8}$, un cycloalkyle en $C_{3-8}$ ou un alkylène en $C_{2-6}$ ;

le radical $R^{23}$ représentant H, un radical alkyle en $C_{1-10}$, un radical aryle ou hétéroaryle ou un radical aryle ou hétéroaryle relié par un groupe alkylène en $C_{1-3}$, ces radicaux aryle et hétéroaryle ne pouvant pas être substitués eux-mêmes par des radicaux aryle ou hétéroaryle,
les radicaux $R^{24}$ et $R^{25}$, identiques ou différents, représentant H, un radical alkyle en $C_{1-10}$, un radical aryle ou hétéroaryle ou un radical aryle ou hétéroaryle relié par un groupe alkylène en $C_{1-3}$, ces radicaux aryle et hétéroaryle ne pouvant pas être substitués eux-mêmes par des radicaux aryle ou hétéroaryle,
ou les radicaux $R^{24}$ et $R^{25}$ signifiant ensemble $CH_2CH_2OCH_2CH_2$, $CH_2CH_2NR^{26}CH_2CH_2$ ou $(CH_2)_{3-6}$, et le radical $R^{26}$ représentant H, un radical alkyle en $C_{1-10}$, un radical aryle ou hétéroaryle ou un radical aryle ou hétéroaryle relié par un groupe alkylène en $C_{1-3}$, ces radicaux aryle et hétéroaryle ne pouvant pas être substitués eux-mêmes par des radicaux aryle ou hétéroaryle,

sous la forme de leurs racémats, de leurs énantiomères ou diastéréomères purs, de mélanges des énantiomères ou diastéréomères en un rapport de mélange quelconque sous la forme représentée ou sous la forme de leurs sels physiologiquement compatibles, ou sous la forme de leurs solvates.

**2.** 4-aminocyclohexanols substitués selon la revendication 1, **caractérisés en ce que** R' et $R^2$ signifient méthyle.

**3.** 4-aminocyclohexanols substitués selon la revendication 1, **caractérisés en ce que** $R^3$ est choisi parmi cycloalkyle en $C_{3-8}$, substitué une ou plusieurs fois ou non substitué ; aryle, substitué une ou plusieurs fois ou non substitué ; hétérocyclyle, saturé ou insaturé, substitué une ou plusieurs fois ou non substitué ; aryle ou cyrloalkyle en $C_{3-8}$ relié par un pont alkyle en $C_{1-3}$, chacun substitué une ou plusieurs fois ou non substitué, ou hétérocyclyle relié par un pont alkyle en $C_{1-3}$, saturé ou insaturé, chacun substitué une ou plusieurs fois ou non substitué.

**4.** 4-aminocyclohexanols substitués selon la revendication 1, **caractérisés en ce que** $R^4$ est choisi parmi cycloalkyle en $C_{3-8}$, aryle ou hétéroaryle, chacun non substitué ou substitué une ou plusieurs fois ; ou $-R^8-L-R^9$.

**5.** 4-aminocyclohexanols substitués selon la revendication 4, **caractérisés en ce que**

$R^8$ est choisi parmi

indolyle, naphtyle, benzofuranyle, benzothiophényle, indanyle, benzodioxanyle, benzodioxolanyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle furanyle, benzofuranyle, pyrazolinonyle, oxopyrazolinonyle, pyrimidinyle, quinolinyle, isoquinolinyle, phtalazinyle ou quinazolinyle, chacun non substitué ou substitué une ou plusieurs fois,

L est choisi parmi

-C(O)-NH-, -NH-C(O)-, -C(O)-O-, -O-C(O)-, -O-, -Sou $-S(O)_2-$,

et/ou $R^9$ est choisi parmi

indolyle, naphtyle, benzofuranyle, benzothiophényle, indanyle, benzodioxanyle, benzodioxolanyle, acénaphtyle, carbazolyle, phényle, thiophényls, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furanyle, benzofuranyle, pyrazolinonyle, oxopyrazolinonyle, pyrimidinyle, quinolinyle, isoquinolinyle, phtalazinyle ou quinazolinyle, chacun non substitué ou substitué une ou plusieurs fois.

**6.** 4-aminocyclohexanols substitués selon la revendication 1, **caractérisés en ce que** $R^4$ est choisi parmi $-CHR^6R^7$, $-CHR^6-CH_2R^7$, $-CHR^6-CH_2-CH_2R^7$, $-CHR^6-CH_2-CH_2-CH_2R^7$.

**7.** 4-aminocyclohexanols substitués selon la revendication 6, **caractérisés en ce que** $R^6$ est choisi parmi

H, alkyle en $C_{1-4}$, saturé ou insaturé, ramifié ou non ramifié, substitué une ou plusieurs fois ou non substitué ; ou $C(O)OR^{10}$

$R^{10}$ étant choisi parmi alkyle en $C_{1-4}$, saturé ou insaturé, ramifié ou non ramifié, substitué une ou plusieurs fois ou non substitué.

**8.** 9-aminocyclohexanols substitués selon l'une quelconque des revendications 6 à 7, **caractérisés en ce que**

$R^7$ est choisi parmi cycloalkyle en $C_3$-$C_8$, aryle ou hétéroaryle, chacun non substitué ou substitué une ou plusieurs fois.

**9.** 4-aminocyclohexanols substitués selon l'une quelconque des revendications 1 à 8, **caractérisés en ce qu'**ils sont choisis dans le groupe suivant :

- la trans-(4-benzyloxy-1-phénylcyclohexyl)diméthylamine,
- la cis-(4-benzyloxy-1-phénylcyclohexyl)diméthylamine,
- la trans-(1-benzyl-4-benzyloxycyclohexyl)diméthylamine,
- la cis-(1-benzyl-4-benzyloxycyclohexyl)diméthylamine,
- la trans-[4-benzyloxy-1-(2-méthylbenzyl)cyclohexyl]diméthylamine,
- la cis-[4-benzyloxy-1-(2-méthylbanzyl)cyclohexyl]diméthylamine,
- la cis-[4-(2-fluorobanzyloxy)-1-phénylcyclohexyl]diméthylamine,
- la cis-[1-benzyl-4-(3-fluorobenzyloxy)cyclohexyl]diméthylamine,
- la cis-[1-benzyl-4-(2-fluorobenzyloxy)cyclohexyl]diméthylamine,
- cis-[1-benzyl-4-(4-fluorobenzyloxy)cyclohexyl]diméthylamine,
- la trans-[4-[2-fluorobenzyloxy)-1-phénylcyclohexyl]diméthylamine,
- la trans-[4-(3-fluorobenzyloxy)-1-phénylcyclohexyl]diméthy-amine,
- la trans-[4-(4-fluorobenzyloxy)-1-phénylcyclohexyl]diméthylamine,
- la trans-[4-(4-fluorobenzyloxy)-1-phénéthylcyclohexyl]diméthylamine,
- la trans-[4-(3-fluorobenzyloxy)-1-phénéthylcyclohexyl]diméthylamine,

sous la forme de leurs racémats, de leurs énantiomères ou diastéréomères purs, de mélanges des énantiomères ou diastéréomères en un rapport de mélange quelconque ; sous la forme représentée ou sous la forme de leurs sels physiologiquement compatibles, ou sous la forme de leurs solvates.

**10.** Médicament contenant au moins un 4-aminocyclohexanol substitué selon l'une quelconque des revendications 1 à 9 sous la forme de ses racémats, de ses énantiomères ou diastéréoméres purs, de mélanges des énantiomères ou diastéréomères en un rapport de mélange quelconque ; sous la forme représentée ou sous la forme de ses sels physiologiquement compatibles, ou sous la forme de ses solvates.

**11.** Médicament selon la revendication 10, **caractérisé en ce que** le médicament contient également un opioïde en plus d'au moins un 4-aminocyclohexanol substitué.

**12.** Utilisation d'un 4-aminocyclohexanol substitué selon l'une quelconque des revendications 1 à 9 sous la forme de ses racémats, de ses énantiomères ou diastéréomères purs, de mélanges des énantiomères ou diastéréomères en un rapport de mélange quelconque ; sous la forme représentée ou sous la forme de ses sels physiologiquement compatibles, ou sous la forme de ses solvates, pour la fabrication d'un médicament pour le traitement de la douleur.

**13.** Utilisation d'un 4-aminocyclohexanol substitué selon l'une quelconque des revendications 1 à 9 sous la forme de ses racémats, de ses énantiomères ou diastéréoméres purs, de mélanges des énantiomères ou diastéréoméres en un rapport de mélange quelconque ; sous la forme représentée ou sous la forme de ses sels physiologiquement compatibles, ou sous la forme de ses solvates, pour la fabrication d'un médicament pour le traitement des états d'anxiété, du stress et des syndromes associés avec le stress, des dépressions, de l'épilepsie, de la maladie d'Alzheimer, de la démence sénile, des dysfonctionnements cognitifs généraux, des difficultés d'apprentissage et

de mémorisation (en tant que nootropique), des phénomènes de sevrage, de l'abus de et/ou de la dépendance à l'alcool et/ou aux drogues et/ou aux médicaments, des dysfonctionnements sexuels, des maladies cardiovasculaires, de l'hypotension, de l'hypertension, des acouphènes, du prurit, de la migraine, de la surdité, du manque de motilité intestinale, des troubles de l'alimentation, de l'anorexic, de l'obésité, des troubles locomoteurs, de la diarrhée, de la cachexie, de l'incontinence urinaire ou en tant que relaxant musculaire, anticonvulsif ou anesthésique ou pour la co-administration lors du traitement avec un analgésique opioïde ou avec un anesthésique, pour la diurèse ou l'antinatriurèse et/ou l'anxiolyse.

**14.** Procédé de fabrication d'un 4-aminocyclohexanol substitué selon la revendication 1, comprenant les étapes suivantes :

a. une cyclohexane-1,4-dione de formule IV protégée avec les groupes $S^1$ et $S^2$ est mise en réaction en présence d'un composé de formule $HNR^{01}R^{02}$ avec un cyanure pour former un dérivé de 1-amino-4-oxo-cyclohexanecarbonitrile N-substitué protégé de formule v ;

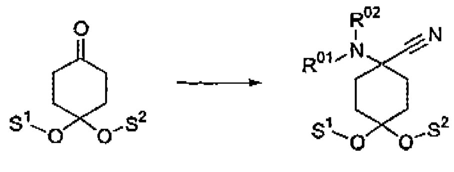

IV V

b. l'aminonitrile de formule v est mis en réaction avec des réactifs métallo-organiques, de préférence des réactifs de Grignard ou d'organolithium, de formule métal-$R^3$, afin de former un composé de formule VI ;

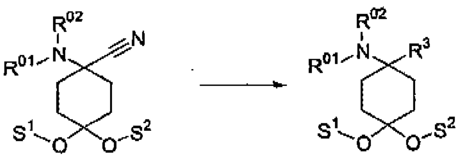

V VI

c. les groupes protecteurs $S^1$ et $S^2$ sont clivés du composé de formule VI afin de former un dérivé de 4-aminocyclohexanone 4-substitué de formule VII

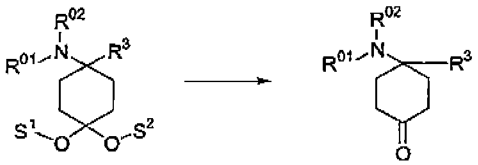

VI VII

d. le dérivé de 4-aminocyclohexanone 4-substitué de formule VII est mis en réaction avec un réducteur tel que par exemple le borohydrure de sodium, le cyanoborohydrure de sodium, le triacétoxyborohydrure de sodium, l'hydrure de lithium-aluminium, l'hydrure de diisobutyl-aluminium, un analogue complexe de ces composés, à des températures comprises entre -70 °C et +110 °C, ou catalysé par un métal noble avec de l'hydrogène, pour former un dérivé de 4-aminocyclohexanol de formule VIII ;

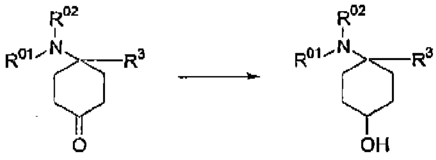

VII VIII

e. le dérivé de 4-aminocyclohexanol 4-substitué de formule VIII est ensuite mis en réaction en présence d'une base inorganique, métallo-organique ou organique avec un bromure, chlorure, iodure, triflate d'alkyle, d'acyle ou d'aryle ou avec un alcane, acide alkylique ou composé aromatique $R^4X$ muni d'un autre groupe partant X, pour former un composé de formule I,

$R^1$, $R^2$, $R^3$ et $R^4$ ayant la signification donnée dans la revendication 1, et
$R^{01}$ et $R^{02}$ étant choisis indépendamment l'un de l'autre parmi H ; H muni d'un groupe protecteur ; alkyle en $C_{1-8}$ ou cycloalkyle en $C_{3-8}$, chacun saturé ou insaturé, ramifié ou non ramifié, substitué une ou plusieurs fois ou non substitué ; aryle ou hétéroaryle, chacun substitué une ou plusieurs fois ou non substitué ; ou aryle, cycloalkyle en $C_{3-8}$ ou hétéroaryle relié par un alkylène en $C_{1-3}$, chacun substitué une ou plusieurs fois ou non substitué.

**15.** Procédé alternatif de fabrication d'un 4-aminocyclohexanol substitué selon la revendication 1, comprenant les étapes suivantes :

a. une cyclohexane-1,4-dione de formule IV protégée avec les groupes $S^1$ et $S^2$ est mise en réaction avec un réducteur tel que par exemple le borohydrure de sodium, le cyanoborohydrure de sodium, le triacétoxyborohydrure de sodium, l'hydrure de lithium-aluminium, l'hydrure de diisobutyl-aluminium, un analogue complexe de ces composés, à des températures comprises entre -70 °C et +110 °C, ou catalysé par un métal noble avec de l'hydrogène, pour former un dérivé de 4-hydroxycyclohexanone protégé de formule XIII ;

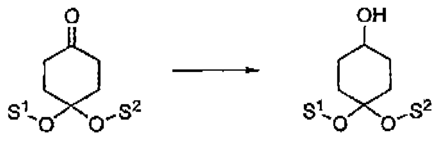

IV XIII

b. qui est ensuite mis en réaction en présence d'une base anorganique, métallo-organique ou organique avec un bromure, chlorure, iodure, triflate d'alkyle ou d'aryle ou avec un alcane ou composé aromatique $R^4X$ muni d'un autre groupe partant X pour former un composé de formule XIV ;

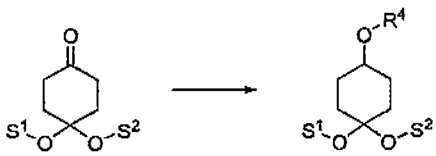

XIII XIV

c. les groupes protecteurs $S^1$ et $S^2$ sont clivés du composé de formule XIV afin de former un composé de formule XV ;

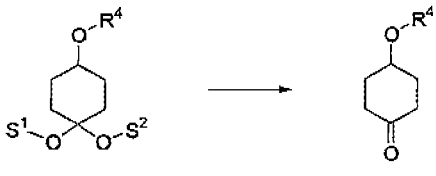

XIV XV

d. le composé de formule XV est mis en réaction en présence d'un composé de formule $HNR^{01}R^{02}$ avec un cyanure pour former un dérivé d'α-aminonitrile de formule XVI ;

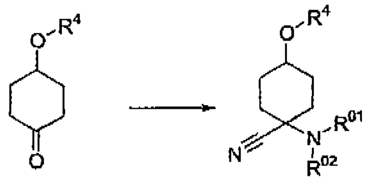

XV XVI

e. le dérivé d'α-aminonitrile de formule XVI est mis en réaction avec des réactifs métallo-organiques de formule métal-$R^3$ pour former un composé de formule I ;

$R^1$, $R^2$, $R^3$ et $R^4$ ayant la signification donnée dans la revendication 1, et
$R^{01}$ et $R^{02}$ étant choisis indépendamment l'un de l'autre parmi H ; H muni d'un groupe protecteur ; allyle en $C_{1-8}$ ou cycloalkyle en $C_{3-8}$, chacun saturé ou insaturé, ramifié ou non ramifié, substitué une ou plusieurs fois ou non substitué ; aryle ou hétéroaryle, chacun substitué une ou plusieurs fois ou non substitué ; ou aryle, cycloalkyle en $C_{3-8}$ ou hétéroaryle relié par un alkylène en $C_{1-3}$, chacun substitué une ou plusieurs fois ou non substitué.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4366172 A
- WO 9614307 A


### In der Beschreibung aufgeführte Nicht-Patentliteratur


- **MEUNIER et al.** *Nature,* 1995, vol. 377, 532-535
- **MOLLEREAU et al.** *FEBS Letters,* 1994, vol. 341, 33-38
- **DARLAND et al.** *Trends in Neurosciences,* 1998, vol. 21, 215-221
- **ARDATI et al.** *Mol. Pharmacol.,* vol. 51, 816-824
- **MATTHES et al.** *Mol. Pharmacol.,* 1996, vol. 50, 447-459
- **VAUGHAN et al.** *Br. J. Pharmacol.,* 1996, vol. 117, 1609-1611
- **CONNER et al.** *Br. J. Pharmacol.,* 1996, vol. 118, 205-207
- **KNOFLACH et al.** *J. Neuroscience,* 1996, vol. 16, 6657-6664
- **REINSCHEID et al.** *Science,* 1995, vol. 270, 792-794
- **HARA et al.** *Br. J. Pharmacol.,* 1997, vol. 121, 401-408
- **MOGIL et al.** *Neurosci. Letters,* 1996, vol. 214, 131-134
- *Neuroscience,* 1996, vol. 75, 333-337
- **JENCK et al.** *Proc. Natl. Acad. Sci. USA,* 1997, vol. 94, 14854-14858
- **SHU et al.** *Neuropeptides,* 1998, vol. 32, 567-571
- **FABER et al.** *Br. J. Pharmacol.,* 1996, vol. 119, 189-190
- **KING et al.** *Neurosci. Lett.,* 1997, vol. 223, 113-116
- **XU et al.** *NeuroReport,* 1996, vol. 7, 2092-2094
- **YAMAMOTO et al.** *Neuroscience,* 1997, vol. 81, 249-254
- **YAMAMOTO ; NOZAKI-TAGUCHI.** *Anesthesiology,* 1997, vol. 87
- **ABDULLA ; SMITH.** *J. Neurosci.,* 1998, vol. 18, 9685-9694
- **SANDIN et al.** *Eur. J. Neurosci.,* 1997, vol. 9, 194-197
- **MANABE et al.** *Nature,* 1997, vol. 394, 577-581
- **NISHI et al.** *EMBO J.,* 1997, vol. 16, 1858-1864
- **POMONIS et al.** *NeuroReport,* 1996, vol. 8, 369-371
- **GUMUSEL et al.** *Life Sci.,* 1997, vol. 60, 141-145
- **CAMPION ; KADOWITZ.** *Biochem. Biophys. Res. Comm.,* 1997, vol. 234, 309-312
- **GUTIÉRREZ et al.** *Society for Neuroscience, Vol 24, 28th Ann. Meeting, Los Angeles,* 07. November 1998, vol. 24
- **KAPISTA et al.** *Life Sciences,* 1997, vol. 60, L 15-21
- **CALO et al.** *Br.J. Pharmacol.,* 2000, vol. 129, 1261-1283
- **A KALIR et al.** *Israel Journal of chemistry,* 1975, vol. 13 (2), 125-136
- **J. VIGNON et al.** *European journal of pharmacology,* 1982, vol. 81 (4), 531-542
- **ARDATI et al.** *Mol. Pharmacol.,* 1997, vol. 51, 816-824